(19) Europäisches Patentamt

European Patent Office

Office européen des brevets

(11) **EP 0 635 027 B1**

## (12) EUROPEAN PATENT SPECIFICATION

(45) Date of publication and mention
of the grant of the patent:
**12.07.2000 Bulletin 2000/28**

(21) Application number: **93907942.2**

(22) Date of filing: **29.03.1993**

(51) Int. Cl.⁷: **A61K 38/00**, C07K 5/10,
C07K 7/06

(86) International application number:
**PCT/GB93/00649**

(87) International publication number:
**WO 93/20102 (14.10.1993 Gazette 1993/25)**

(54) **PEPTIDE T AND RELATED PEPTIDES IN THE TREATMENT OF INFLAMMATION, INCLUDING MULTIPLE SCLEROSIS**

T-PEPTID UND DAMIT VERWANDTE PEPTIDE IN DER BEHANDLUNG VON ENTZÜNDUNGEN EINSCHLIESSLICH DER MULTIPLEN SKLEROSE

PEPTIDE T ET PEPTIDES ASSOCIES DESTINES AU TRAITEMENT DES INFLAMMATIONS, NOTAMMENT LA SCLEROSE EN PLAQUES

(84) Designated Contracting States:
**AT BE CH DE DK ES FR GB GR IE IT LI LU MC NL PT SE**

(30) Priority: **27.03.1992 US 858832
14.05.1992 DK 64592
17.07.1992 US 915118
09.12.1992 US 987674**

(43) Date of publication of application:
**25.01.1995 Bulletin 1995/04**

(60) Divisional application:
**99101349.1 / 0 960 886**

(73) Proprietor:
**ADVANCED IMMUNIT T,INC.
Stony Brook, NY 11790 (US)**

(72) Inventors:
• **ANDERSEN, Anders, Jörgen
DK-2980 Kokkedal (DK)**
• **ASTON, Roger
Malmesbury, Wiltshire SN16 9HW (GB)**
• **CARLEN, Peter, Louis
Toronto, Ontario M5N 1M3 (CA)**
• **DOOB, Penelope, Reed
Toronto, Ontario M4J 3HZ (CA)**
• **MacFADDEN, Douglas, Kevin
Toronto, Ontario M4J 1K9 (CA)**
• **PHIPPS, David, James
Toronto, Ontario M6H 3M7 (CA)**
• **RATHJEN, Deborah
Thornleigh, NSW 2120 (AU)**
• **WIDMER, Fred
Ryde, NSW 2112 (AU)**

(74) Representative:
**Woods, Geoffrey Corlett et al
J.A. KEMP & CO.
Gray's Inn
14 South Square
London WC1R 5LX (GB)**

(56) References cited:
**EP-A- 0 249 390          WO-A-88/09338
WO-A-89/12067          WO-A-92/19257
FR-A- 2 603 107**

• **FILE SERVER STN KARLSRUHE,FILE MEDLINE ABSTRACT NO.89131477**
• **FILE SERVER STN KARLSRUHE,FILE MEDLINE ABSTRACT NO.77209062**
• **BIOCHEMICAL AND BIOPHYSICAL RESEARCH COMMUNICATIONS vol. 171, no. 2, 14 September 1990, NEW YORK,USA pages 596 - 602 COTELLE ET AL 'CONFORMATIONAL STUDY OF THE THREONINE-RICH C-TERMINAL PENTAPEPTIDE OF PEPTIDE T'**

• GASTROENTEROLOGY vol. 96, no. 5(2), 1989, NEW YORK,USA page A471 SIBUG ET AL 'ACUTE AND CHRONIC EFFECTS OF MAST CELL MEDIATORS ON INTESTINAL EPITHELIAL FUNCTION'

• BIOCHEMICAL AND BIOPHYSICAL RESEARCH COMMUNICATIONS vol. 184, no. 2, 30 April 1992, NEW YORK,USA pages 1125 - 1132 URGE ET AL 'CHEMICAL GLYCOSYLATION OF PEPTIDE T AT NATURAL AND ARTIFICIAL GLYCOSYLATION SITES STABILIZES OR REARRANGES THE DOMINANT REVERSE TURN STRUCTURE' cited in the application

**Description**

The present invention relates to the treatment or prevention of inflammatory bowel disease, whether caused by bacteria, viruses and/or other infective agents, opportunistic infections (which may be consequent on an immunodepressed state, for example resulting from cancer or therapy, particularly cytotoxic drug therapy or radiotherapy) autoimmmunity or otherwise. The invention also relates to pharmaceutical compositions useful in such treatment and/or prevention and to certain active peptides *per se.*

Inflammatory bowel disease (IBD) is a chronic inflammatory condition that fulfils some of the criteria of an autoimmune disease (Snook, *Gut* **31** 961-963 (1990). Inflammation and tissue damage involves the recruitment and activation of neutrophils, macrophages and lymphocytes (MacDermott *et al,* Adv. *Immunol.* **42** 285-328 (1998) which generate cytokines and proinflammatory molecules such as prostaglandins and leukotrienes (MacDermott, *Mt. Sinai J. Med.* **57** 273-278 (1990). As a result of chronic activation of immunocompetent cells, IL-1, IL-6 (Starter, *Immunol.* Res. **10** 465-471 (1991); Fiocchi, *Immunol.* Res. **10** 239-246 (1991) and TNF-$\alpha$ (MacDermott, *Mt. Sinai J.* Med. **57** 273-278 (1990) are all elevated in IBD patients.

Drugs used to treat IBD include anti-inflammatory agents such as sulphasalazine (5-ASA), corticosteroids, cyclosporin A and azathioprine (Hanauer, *Scand. J. Gastroenterol.* **25** (Suppl. 175) 97-106 (1990); Peppercorn, *Annal. Intern. Med.* **112** 50-60 (1990). Experimentally, anti-CD4 monoclonal antibodies have been used to successfully treat ulcerative colitis (Emmrich *et al Lancet* **338** 570-571 (1991).

Marcusson and Wetterberg (1989), Acta Derm. Venereol., 69(1), 86-8 describes the use of peptide T in the treatment of psoriasis and psoriatic arthritis.

WO-A-89/12067 discloses that peptides which inhibit binding of human immunodeficiency virus (HIV) to receptor sites on the cell surface have been shown to be useful as agents for treatment of psoriasis and neuropsychiatric disorders, including memory deficit and mood disorders.

FR-A-2603107 discloses compositions comprising a therapeutically effective dose of a monoclonal antibody which reacts with one or more neutralising regions of HIV, or a plurality of blocking peptides capable of attenuating HIV infection and/or monoclonal antibodies which react with the epitopes of such peptides; and a pharmaceutically acceptable carrier. The compositions can be used to treat HIV infection.

WO-A-92/19257 discloses a method of treating chronic fatigue syndrome not associated with an HIV infection. In the method patients are administered a pharmaceutically acceptable carrier together with (1) a neuropsychiatrically effective amount of a linear peptide of formula (I): $R^a$-Ser-Thr-Thr-Thr-Asn-Tyr-$R^6$, wherein $R^a$ is Ala. D-Ala or Cys-Ala- and $R^6$ is Thr, Thr-amide, Thr-Cys or Thr-Cys-amide, or a derivative of the peptide or a physiologically acceptably salt thereof; or (2) a neuropsychiatrically effective amount of a liner peptide of formula (II): $R^1$-$R^2$-$R^3$-$R^4$-$R^5$, wherein $R^1$ is X-Y or Y when Y is Thr-, Ser-, Asn-, Leu-, Ile-, Arg- Glu-, and X is Cys; $R^2$ is Thr-, Ser- or Asp; $R^3$ is Thr, Slr, Asn, Arg, Gln, Lys or Trp; $R^4$ is Tyr; and $R^5$ is Z-X or Z wherein Z is an amino acid and X is Cys, or a derivative of the peptide or a physiologically acceptable salt thereof; or (3) a neuropsychiatrically effective amount of a linear peptide of formula (III): $R^x$-$R^2$-$R^3$-$R^4$-$R^y$, wherein $R^x$ is Ala-$R^1$, D-Ala-$R^1$ or X-Ala-$R^1$ wherein X, $R^1$,$R^2$·$R^3$,$R^4$ are as defined above, and $R^y$ is Thr-, Thr-amide or Thr-Cys, or a derivative of the peptide or a physiologically acceptable salt thereof.

The present invention deals with the identification of a group of peptides that alleviate the inflammatory response in inflammatory bowel disease. Peptides useful in the present invention do not necessarily interfere directly with the pathogenic mechanisms of the disease-causing component. As will be described below in the experimental data, the mechanism whereby these peptides can alleviate the symptoms of these diseases is dependent on their capability of modulating the production and effect of cytokines produced by activated cells of the immune system. The modulation of cytokines may not be limited to TNF but may also be valid for a whole range of interleukins, for example from interleukin-1 to interleukin-10. The data presented are at present not direct evidence, but rather a powerful indirect model. Thus, the model uses one of the most powerful inflammatory compounds known, LPS, which binds to receptors on neutrophils, monocytes and macrophages; these cells consequently become activated and start production of IL-1 and TNF, among other cytokines, thus starting the inflammatory cascade. One parameter used to measure this effect of LPS is the concentration of blood glucose, which normally decreases on exposure to TNF or LPS. From what is known in the literature about the mechanism of Peptide T at a cellular level, it is therefore highly surprising that Peptide T and its analogues are able significantly to reduce the negative effects of LPS. LPS normally combines with LPS-Binding Protein (LBP) and exerts its dramatic effect through the CD14 receptor. In the literature up to date, the peptides useful in this invention have only been connected to the CD4 receptor, which is not believed to be involved in the primary inflammatory response associated with cytokines, such as TNF, or LPS.

It is important that particular compounds have been found which are useful in treating inflammatory bowel disease where it appears that these compounds interact in some manner with CD4 receptors of immune system cells. The compounds relate, as indicated above, to Peptide T and its various derivatives. It was originally thought that such compounds had no effect on the immune system other than being very useful in blocking attachment of HIV virus to CD4 receptor cells (Ruff *et al,* IV International Conference on AIDS, Stockholm June 1988).

Originally, many of the peptides useful in the invention were described as being effective in the prevention of infection and replication of HIV *in vitro;* see EP-A-0249390, EP-A-0249394 and WO-A-8809338. All compounds disclosed in these specifications are useful for the present invention. The original peptide has its basic point of origin in the octapeptide Ala-Ser-Thr-Thr-Thr-Asn-Tyr-Thr. It is called Peptide T because 50% of the amino acid residues are threonines. This peptide has been identified from a subregion of the human immune deficiency virus (HIV) external glycoprotein molecule gp120, which is responsible for binding to any cell carrying the CD4 molecule and, in particular, helper lymphocytes, microglial cells in the CNS, monocytes and dendritic cells. Binding occurs via specific attachment of gp120 to the CD4 molecule. Treating individuals infected with HIV with this peptide and its derivatives, which are described below, consequently has the effect of potentially inhibiting binding of the whole virus or the neurotoxic gp120 molecule to the cell receptor CD4. In this way, the cell is protected from infection, and so the virus, being unable to replicate, is destroyed by the immune defence.

A first aspect of the invention provides use of a linear or cyclic peptide of General Formula 1:

I-A-B-C-D-E-F-G-H-II      (General Formula 1, SEQ ID NO 1)

wherein

A is Ala, Gly, Val, Ser, Thr or absent,
B is Ala, Gly, Val, Ser, Thr or absent,
C is Ser, Thr or absent
D is Ser, Thr, Asn, Glu, Arg, Ile, Leu or absent,
E is Ser, Thr, Asp or absent
F is Thr, Ser, Asn, Arg, Gln, Lys, Trp or absent,
G is Tyr or absent,
H is Thr, Arg, Gly, Met, Met(O), Cys, Thr, Gly or absent,
and
I is Cys or absent,
II is Cys, an amide group, substituted amide group, an ester group or absent,

and wherein the peptide comprises the amino acid sequence

-Thr-Thr-Asn-Tyr-Thr-      (SEQ ID NO 3).

one or more of the amino acids being substituted by a monomeric or polymeric carbohydrate or derivative thereof, such substitution being accomplished through hydroxyl and/or amino and/or amido groups of the amino acids,
or a pharmaceutically acceptable salt thereof, in the manufacture of a medicament for treating or preventing inflammatory bowel disease.

Each of the amino acids referred to in General Formula 1 may be in the L- or D- stereoisomeric configuration and candidates for H may be esterified or amidated.

It is appreciated that peptides may have at both ends of the core sequence Thr-Thr-Asn-Tyr-Thr additional amino acid residues, some of which are represented by General Formula 2:

X-Ser-Thr-Thr-Thr-Asn-Tyr-Y      (General Formula 2)

wherein X is an amino acid terminal residue selected from Ala and D-Ala and Y is a carboxy terminal residue selected from Thr and Thr-amide.

The term Peptide T is used throughout the specification to reference, unless the context otherwise requires, peptides of General Formula 2. It is therefore intended that Peptide T encompass all of the compounds of General Formula 2 where it is understood that all such compounds are variants of the normally understood octapeptide T, also referred to as prototype Peptide T, of the particular formula D-Ala-Ser-Thr-Thr-Thr-Asn-Tyr-Thr-amide.

The invention may be useful in both clinical (human) and veterinary medicine. The invention therefore has application for treating or preventing inflammatory bowel disease by administering to a human or other animal subject, for example on a repeated basis, a peptide of General Formula 1. The peptide will generally be administered in an effective, non-toxic amount or in such an amount that strikes an acceptably balance between efficacy and toxicity, having regard to the circumstances of the case.

In a preferred embodiment the invention provides use of a linear or cyclic peptide which is

1. D-Ala-Ser-Thr-Thr-Thr-Asn-Tyr-Thr-NH$_2$;

2. Ala-Ser-Thr-Thr-Thr-Asn-Tyr-Thr        (SEQ ID NO 5);

3. D-Ala-Ser-Thr-Thr-Thr-Asn-Tyr-Thr;

4. D-Ala-Ala-Ser-Ser-Ser-Asn-Tyr-Met;

5. Thr-Asp-Asn-Tyr-Thr        (SEQ ID NO 6);

6. Thr-Thr-Ser-Tyr-Thr        (SEQ ID NO 7);

7. Thr-Thr-Asn-Tyr-Thr        (SEQ ID NO 8);

8. D-Thr-Thr-Tyr-D-Thr;

9. D-Ala-Ser-D-Thr-Thr-D-Thr-Asn-Tyr-D-Thr-NH$_2$; or

10. D-Ser-Ser-D-Thr-Thr-D-Thr-D-Thr-Thr-Tyr-D-Thr-NH$_2$;

or a derivative or salt thereof in the manufacture of a medicament for treating or preventing inflammatory bowel disease.

In particular in the use the peptide may be D-Ala-Ser-Thr-Thr-Thr-Asn-Tyr-Thr-NH$_2$, or a derivative or salt thereof.

Quite often it may be an advantage to have the amino terminal amino acid as a D-stereoisomer, to protect the molecule from degradation from aminopeptidases; alternatively or additionally, the carboxy terminal amino acid may be an amino acid amide to protect the molecule from degradation from carboxypeptidases. In this connection, compounds 5, 6 and 7, listed above, include analogues with D-Thr as the amino terminal residue and/or an amide derivative at the carboxy terminal.

Typically in the peptide at least one of the amino acids is a substituted N-alkyl amino acid. Suitably alkyls include (C$_1$-C$_4$) alkyl, e.g. methyl or ethyl.

Typically in the peptide at least one of the hydroxyl groups on a Ser, Thr or Tyr residue is derivatised into an ester or ether compound.

Any (optionally substituted) alkyl ester or ether may be formed, such as (C$_1$-C$_4$) alkyl, aryl or aryl (C$_1$-C$_4$) alkyl esters, ethers, thioesters and thioethers, for example phenylester, benzylether or thiophenol ethylester. Preferred ethers are methyl, ethyl and propyl ethers and preferred esters are methyl, ethyl and propyl esters.

The hydroxyl side chains of the amino acids Ser, Thr and/or Tyr and the amido groups of the amino acids Asn and/or Gln may be substituted with different carbohydrates or derivatives of carbohydrates,

In one embodiment in the peptide at least one of the amino acids is substituted with a monomeric or polymeric carbohydrate, or a derivative thereof, the substitution(s) being accomplished through hydroxyl- and/or amino- and/or amido-groups of the amino acids.

Linear peptides useful in this invention may be prepared by any suitable process, such as conventional solid phase peptide synthetic techniques; see "Solid Phase Peptide Synthetic Techniques", 2nd ed, J.M. Stewart, J.D. Young, Pierce Chemical Company, 1984, ISBN: 0-935940-03-0. A frequently used solid phase method is the Merrifield technique. Another possibility is solution phase techniques. The preferred peptide, prototype Peptide T, is readily obtainable from Carlbiotech A/S, Copenhagen, Denmark.

Cyclic peptides useful in the invention may be prepared by known techniques, such as, for example, described in Y.Hamada in *Tetrahedron Letters,* **26** 5155 (1985). Cyclic peptides may be established in the form of a disulphide bridge between two Cys residues and/or by reacting the carboxy terminal amino acid residue with the amino terminal residue and/or by reacting the amino terminal residue with for example the γ-carboxyl group of Glu, when Glu is at position D.

Carbohydrate derivatives may be prepared by methods known in the art. The invention also provides any of the peptides of the invention mentioned above conjugated to a peptide, protein or other appropriate carrier and/or an excipient.

In another aspect the invention provides a linear or cyclic peptide of General Formula 1:

I-A-B-C-D-E-F-G-H-II        (General Formula 1, SEQ ID NO 1)

wherein

A is Ala, Gly, Val, Ser, Thr or absent,

B is Ala, Gly, Val, Ser, Thr or absent,

C is Ser, Thr or absent

D is Ser, Thr, Asn, Glu, Arg, Ile, Leu or absent

E is Ser, Thr, Asp or absent

F is Thr, Ser, Asn, Arg, Gln, Lys, Trp or absent,

G is Tyr or absent,

H is Thr, Arg, Gly, Met, Met(O), Cys, Thr, Gly or absent,

I is Cys or absent,

II is Cys or absent,

and wherein the peptide comprises the amino acid sequence

-Thr-Thr-Asn-Tyr-Thr-          (SEQ ID NO 3).

one or more of the amino acids being substituted by a monomeric or polymeric carbohydrate or derivative thereof, such substitution being accomplished through hydroxyl and/or amino and/or amido groups of the amino acids,
with the proviso that the peptide is not glycosylated prototype Peptide T, Thr-Thr-Asn(GlcNAc)-Tyr-Thr or Thr (GalNAc)-Thr-Thr-Asn-Tyr-Thr;
or a pharmaceutically acceptable salt thereof.

Glycosylated Peptide T is disclosed in *Urge et al., Biochem. Biophys; Res. Comms.* **184** (2) 1125-1132 (1992), published 30 April 1992, but the utility of the present invention is neither disclosed nor suggested.

Preferred features of this aspect of the invention are as for the first aspect.

Peptides useful in the invention may be administered as a composition in conjunction with a pharmaceutically acceptably carrier.

In this way the peptides can be used in pharmaceutical compositions and compositions of matter for treating and preventing inflammatory bowel disease

The peptides or peptide formulations may be used alone or in combination with any other pharmaceutically active compound, such as an anti-infective agent, for example an antibiotic and/or antiviral agent and/or antifungal agent, or another pharmaceutically active compound, such as an anti-neoplastic agent.

The peptides may be administered orally, bucally, parenterally, topically, rectally, vaginally, by intranasal inhalation spray, by intrapulmonary inhalation or in other ways.

In particular, the peptides according to the invention may be formulated for topical use, for inhalation with spray or powder, for injection (for example subcutaneous, intramuscular, intravenous, intra-articular or intrcisternal injection), for infusion or for oral administration and may be presented in unit dose form in ampoules or tablets or in multidose vials or other containers with an added preservative. The compositions may take such forms as suspensions, solutions, or emulsions or gels in oily or aqueous vehicles, and may contain formulatory agents such as suspending, stabilising and/or dispersing agents. Alternatively, the active ingredient may be in powder and/or lyophilised form for direct administration or for constitution with a suitable vehicle (eg sterile, pyrogen-free water, normal saline or 5% dextrose) before use. The pharmaceutical compositions containing peptide(s) may also contain other active ingredients such as antimicrobial agents, or preservatives.

The compositions may contain from 0.001-99% (w/v or, preferably, w/w) of the active material. Peptide T obtainable from Carlbiotech A/S is usually formulated and packaged in a sterile manner in 5% dextrose solution in multi-dose vials. It will be appreciated that the peptide may be packaged in other carriers, such as saline. Preferably, the concentration of peptide in each dose is in the order of 8.5 mg/ml for subcutaneous injection in one ml doses.

The compositions are administered in therapeutically or prophylactic effective doses, ie 0.05-10000 mg of peptide per day, in particular 5-1000 mg per day. Very large doses may be used as the peptide according to the invention is non-toxic. However, normally this is not required. The dose administered daily of course depends on the degree of inflammation and inflammatory response.

For administration by injection or infusion of the composition, the daily dosage, as employed for treatment of adults of approximately 70 kg of body weight, will often range from 0.2 mg to 20 mg of active material which may be administered in the form of 1 to 4 doses over each day, such dosage ranges depending upon the route of administration and the condition of the patient.

Compositions as described above may be prepared by mixing or otherwise bringing into association the ingredients.

The efficacious results in the use of the above compounds is thought to be due, without being limited to any particular theory, to the immunosuppressive activities of these compounds in chronic inflammatory states.

In order to provide a guideline for the administration of and insight into the use of the peptides according to the invention and the formulation of the compositions, the following is offered as a guide based on the extensive work already conducted in the use of peptide T for treating HIV infection.

Peptide T is an octapeptide homologous to a region of gp120, an HIV envelope glycoprotein, and to human vasoactive intestinal peptide (VIP). It was originally developed by Pert *et al* (EP-A-0249394), to block the binding of gp120 (an HIV envelope glycoprotein) and thus also block binding of HIV to CD4, the specific membrane-bound vial receptor, thereby blocking internalisation of the virus into the cell - a process necessary for viral replication. The CD4 molecule necessary for the entry of HIV into cells has been localised on the surface of lymphocytes, macrophages, microglial cells, neurons and numerous other cells. Binding of HIV to the CD4 receptor has been demonstrated to effect viral entry; and binding of free (non-viral related) gp120 has resulted in neuronal toxicity in both *in vitro* and *in vivo* studies.

The efficacy of Peptide T in reversing signs of HIV-induced dementia has been demonstrated in both the Peptide T Phase I clinical trial at the University of Southern California in Los Angeles and in the Phase II clinical trial at the Fenway Clinic in Boston. Both studies have demonstrated improvement in the HIV- induced neurocognitive impairment in patients with AIDS.

To date, the Peptide T/gp120/VIP homology has been used to explain at least two possible mechanisms of action of Peptide T. Firstly, that it competitively binds to CD4 (the known receptor for HIV) on human cell surfaces and competes with both HIV and gp120 for binding sites.

The binding of Peptide T and its analogues of General Formula 1, or more particularly General Formula 2, to CD4 could produce a blocking effect to prevent the binding of any other molecule capable of binding to that receptor; alternatively, or in addition, the binding of Peptide T to CD4 could induce a reaction similar to that caused by the endogenous ligand.

CD4 is the differentiation antigen that defines the T lymphocyte subgroup of helper/inducer cells, but it is also present on a wide variety of cells including neurons, activated macrophages and B cells. CD4 is the predominant receptor for HIV and was originally thought to be necessary for cellular infection. Using the monoclonal antibody OKT4, Pert *et al* (*Proc. Natl. Sci. USA* **83** 9254-9258 (1986); Pert *et al* (*Clin. Neuropharmacol.* **9**(4) S198 (1986)) demonstrated the presence of this antigen throughout the human CNS and showed that it is present in highest concentration in the dentate gyrus, hippocampus, amygdala and deep cortex. This distribution was found to be similar to that found in other higher mammals. Peptide T and similar analogues were found to inhibit the binding of radiolabelled gp120 to rat hippocampal membranes and to do so in 0.1 nM concentrations.

Using Peptide T and the same analogues, Pert *et al* (*Proc*. *Natl. Sci. USA* **83** 9254-9258 (1986); Pert *et al* (*Clin. Neuropharmacol.* **9**(4) S198 (1986)) were able to demonstrate a reduction in the detectable levels of HIV reverse transcriptase when these peptides were present in an assay of HIV infectivity. A ninefold reduction of reverse transcriptase took place at 100 nM concentrations of Peptide T.

Since gp120 is not identical in all isolated strains of HIV, a comparison was made with nine different HIV isolates Pert *et al* (*Clin. Neuropharmacol.* **9**(4) S198 (1986)). Significant homology was found between the isolates examined and Peptide T when comparison was made with the core pentapeptide, (*Ruff et al. FEBS Lett.* **211** 17-22 (1987); Brenneman *et al, Nature,* **335** 639-642 (1988); Brenneman *et al, Drug Dev. Res.* **15** 361-369 (1988); Komisaruk *et al*, *Annals of the NY Acad. Sci.* **527** 650-654 (1988); Buzy *et al, The Lancet* 22 July, 226-227 (1989)). This comparison has now been extended to over twenty isolates.

The inhibition of gp120 and HIV binding to CD4, as well as the demonstration of reduced infectivity of HIV in the presence of Peptide T and its analogues, provides one possible mechanism of action to explain the clinical effects of Peptide T. In this regard, Peptide T in sufficient concentration may prevent new cellular infection with HIV. Initial research in this area was focused on the CNS for two reasons: a high concentration of CD4 molecules was found on neurons and one of the major effects of HIV infection is the development of neurocognitive dysfunction. These fats are particularly important given that Peptide T is transported from the blood to the brain by an active, saturable transport system, while its exit is by diffusion only, (Barrera *et al, Brain Res. Bull.* **19** 629-633 (1987)).

Although it is well accepted that HIV can infect not only lymphocytes but also neurons, it is difficult to ascribe the neurologic dysfunction seen in HIV patients to active CNS HIV infection, since only a small number of neurons are actively infected. It has been suggested that the neurological deficits seen in HIV infection may occur not only as a result of infection but also as a result of a viral "toxin", such as gp120. Brenneman *et al, Nature* **335** 639-642 (1988)) found that purified gp120 from two isolates as well as recombinant gp120 produced significant neuronal cell death in cultures of mouse foetal hippocampal neurons. Neurotoxicity could be reduced by pretreatment with antibody to CD4 and was completely eliminated by VIP. Since mouse neurons cannot be infected with HIV, it is evident that neurotoxicity is gp120-induced and is not a result of viral entry or replication.

VIP (66,67,68,69: TDNYT) and the core peptide (61-65: TTNYT) share the homologous sequence that binds CD4, and that is also found in isolates of the much larger gp120. Peptide T, when used in the same mouse hippocampal neuronal culture system, completely, antagonised the gp120-induced neurotoxicity, Brenneman *et al, Drug Dev. Res.* **15** 361-369 (1988)). In addition, CSF from a patient with AIDS dementia produced substantial neurotoxicity in this system (44-49% killing at 1:100,000 dilution). This effect was inhibited by Peptide T, Buzy *et al, The Lancet,* July 22, pp 226-227, (1989)). Normally gp120 is produced in vast excess of amounts required for viral replication; this excess gp120 may exert a neurotoxic effect far out of proportion to the number of microglial cells or neurons actively infected with HIV.

Peptide T may act as an agonist in addition to or even in the absence of its neuroprotective effects against viral infection and neurotoxicity. Direct agonist activity has been demonstrated in two ways. *Ruff et al,* (*FEBS Lett.* **211** 17-22 (1987)) showed that Peptide T and two analogues were potent agonists of human monocyte chemotaxis. Their rank order potency as chemotactic agents corresponded to their relative ability to inhibit both gp120 binding and HIV T cell infectivity.

As a further demonstration of the agonist activity of Peptide T, both Peptide T and VIP exert their cellular effects via the regulation of protein kinase C: Zorn et *al, The Endoc. Society.* Abstract (1989). Agonist activity of Peptide T is thus implied by the production of a transmembrane signal that can influence the regulation of protein kinase C. It has also been demonstrated in six individual experiments, as part of the present invention, that Peptide T can down-regulate the enzyme p56[lck], a tyrosine kinase linked to the cytoplasmic portion of membrane-bound CD4, thus implying that the binding of Peptide T to the CD4 molecule can produce a transmembrane signal.

Further evidence of Peptide T's potential VIP-like agonist activity is provided by results from experimental testing of the hypothesis of Komisaruk *et al, Annals of* the *NY Acad. Sci.* **527** 650-654 (1988)) that VIP released from pelvic nerve terminals into the spinal cord can produce analgesia. Knowing that naloxone-independent analgesia produced by administration of VIP to the periaqueductal grey matter in rats had been shown, the investigators administered VIP directly to rat spinal cord and measured the pain threshold to distal noxious stimuli to test the hypothesis. Spinal administration of VIP produced analgesia as measured by the tail-flick latency response and the tail-shock induced vocalisation test by action on both opiate and non-opiate modulated pain pathways, Komisaruk *et al, Annals of the NY Acad. Sci.* **527** 650-654 (1988)).

The existence of clinical benefits from the administration of Peptide T to humans has been suggested in all studies to date: in HIV disease, by the Pilot Swedish Study, the USC Phase I and Fenway/CRI studies, and the Toronto Western Hospital Compassionate Use Program involving 51 patients; in psoriasis and other medical conditions, in case reports from Sweden (Marcussion, Lazega *et al,* 1989-9, and Marcusson and Wetterberg, 189-10) and in 8 patients with psoriasis or other medical conditions in Toronto.

Neurocognitive improvement found in HIV positive patients and improvement in constitutional symptoms in both HIV positive and HIV negative patients may well depend primarily on Peptide T's VIP-like neurotrophic and agonist effects, as well as the anti-inflammatory and anti-TNF effects discussed below.

Peptide T appears to act as an agonist and as a blocker of CD4-mediated immune function rather than as an antiviral drug.

Not wishing to be bound by any particular theory with respect to the use of peptides for the treatment of symptoms and diseases associated with chronic immune activation, and inview of the above guidelines and discussions in relation to the use of various peptides of General Formula 2 and their analogues in the treatment of HIV, it is hypothesised that there is an immunomodulatory effect of the Peptide T binding to CD4. Such effect is demonstrated in the following examples where Peptide T has been found to inhibit mitogen induced proliferation of peripheral blood mononuclear cells (PBMC) at picomolar and lower concentrations. We have found that Peptide T allowed PBMC to proliferate in response to mitogen, but at a reduced level compared to the growth of PBMC in its absence. Pre-incubation of PBMC with Peptide T for less than 30 minutes had no effect on mitogen stimulation. However, exposure of PBMC to Peptide T for 2 hours followed by washing of the cells before exposure to the mitogen resulted in inhibition of proliferation similar to that seen when cells are incubated in the presence of both Peptide T and mitogen. It was also found that Peptide T did not significantly affect the growth of PBMC cultured in the absence of mitogen. It is therefore thought that Peptide T is able to suppress the normal proliferative response of PBMC to non-CD4 associated proliferation signals.

Now that the effectiveness of the peptides of General Formula 1, and particularly those of General Formula 2, in treating symptoms and diseases associated with chronic immune activations or chronic inflammation has been discovered, the following is suggested as hypothesis of the mechanism of action of Peptide T and, therefore, why the compounds useful in the invention are effective.

Peptide T binds to CD4. It has been established in the following tests that Peptide T inhibits mitogen and MLR induced lymphocyte proliferation. It is therefore thought that Peptide T may serve as an immunomodulatory drug which would down-regulate the enhanced immune response occurring in the chronic presence of antigen or for other reasons mentioned and hence reduce chronic inflammation.

In accordance with various embodiments of this invention and in view of the above guidelines gained from the use of peptides of General Formula 1, and particularly those of General Formula 2, in the treatment of HIV, similar doses of Peptide T and its analogues can be administered to humans or other animals for purposes of treating inflammatory bowel disease.

The invention will now be illustrated by the following non-limiting examples. The examples refer to the accompanying drawings, in which:

FIGURE 1 is referred to in Example 1 and shows the effect of Peptide T on TNF-induced tissue factor expression on human umbilical vein endothelial cells.

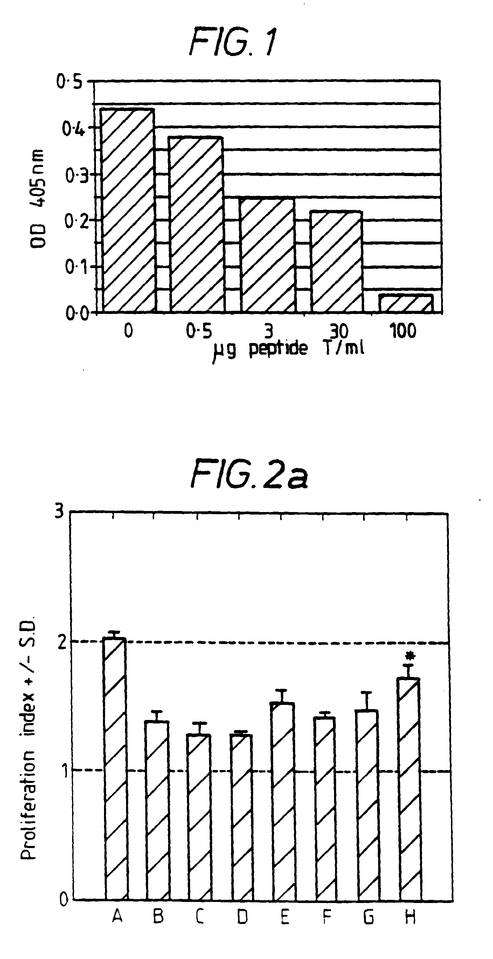

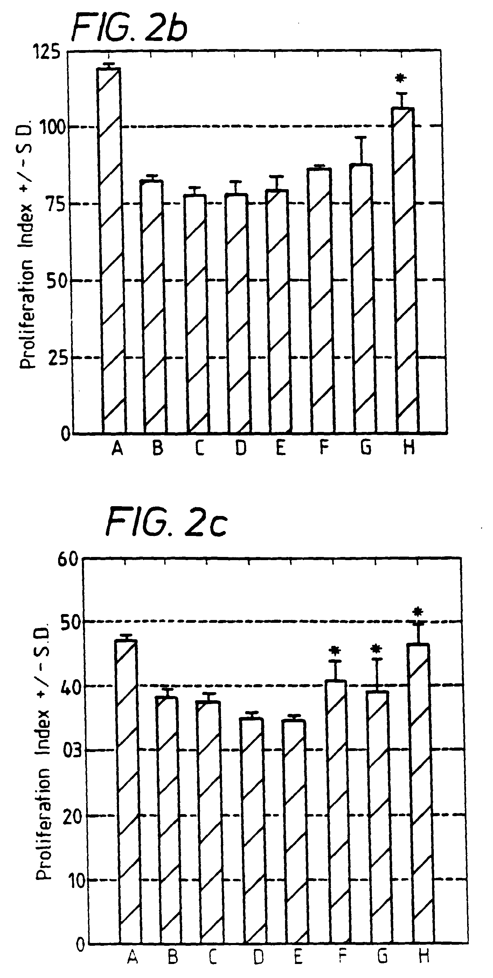

FIGURES 2a, 2b and 2c relate to Example 8 and show that Peptide T was able to inhibit lympho-proliferation in response to mitogens. $10^5$ PBMC were cultured in the presence of PWM (2a), Con A (2b) or PHA (2c) with dilutions of Peptide T as follows: A: no Peptide T; B: $10^{-5}$M; C: $10^{-7}$M, D: $10^{-9}$M; E: $10^{-11}$M; F: $10^{-13}$M; G: $10^{-15}$M; H: $10^{-17}$M Cultures were incubated for 5 days, pulsed with $^3$H-thymidine and the amount of incorporated radioactivity converted into proliferation index as described in Example 17. Asterisk (*) indicates the difference in proliferation; ± is statistically insignificant (p>0.05).

FIGURE 3 relates to Example 8 and shows that Con A-induced lymphocyte proliferation in the presence of Peptide T parallels, but at a reduced rate, growth in the absence of Con A. $10^5$PBMC were cultured in the presence of Con A for 8 days. Cultures were pulsed and harvested daily and the degree of lymphoproliferation expressed as counts per minute (CPM) of incorporated $^3$H-thymidine. Peptide T (PT, $10^{-9}$M), was added concurrently with Con A.

FIGURE 4 relates to Example 9. In the presence of non-mitogen stimuli such as recall antigens (measles, mumps, rubella vaccine: MMR), superantigens such as *Staphylococcal* enterotoxins SEB and TSST-1, and antibodies to CD3 (anti-CD3) PBMC will proliferate as indicated by a proliferation index greater than 1 (-Peptide T). The presence of Peptide T (+ Peptide T) added on the first day of this six day culture had no effect on the growth of PBMC in response to these stimuli.

FIGURE 5 also relates to Example 9. When PBMC from one person (A) are cultured in the presence of PBMC from another person (B) they will proliferate [(A+B(P-PT)] as a result of recognizing foreign antigens (alloantigens) on the cell surface. In the presence of Peptide T [A+B(+PT)] alloresponsive cells are still able to proliferate.

FIGURE 6 also relates to Example 9. Molt-4 is a spontaneously growing human malignant T cell line. In the presence of PBMC growth is inhibited due to killing of-Molt-4 by PBMC (PBMC/Molt-4-PT). In the presence of Peptide T added on the first day of a four day culture, PBMC mediated killing of Molt-4 remained unchanged (PBMC/Molt-4+PT).

FIGURE 7 relates to Example 10, in which is given the sequence of steps involved in this cytotoxicity assay. Activated macrophages are able to kill K562 target cells int he absence of Peptide T (noPT). If Peptide T is added to macrophage cultures concurrently with the activators (PTstep1) it is unable to affect cytotoxicity. If Peptide T is added to activated macrophage cultures concurrently with the target cells (PTstep2) cytotoxicity is inhibited. Altering the timing of addition of Peptide T does not alter the Peptide T- mediated inhibition of macrophage-mediated cytotoxicity.

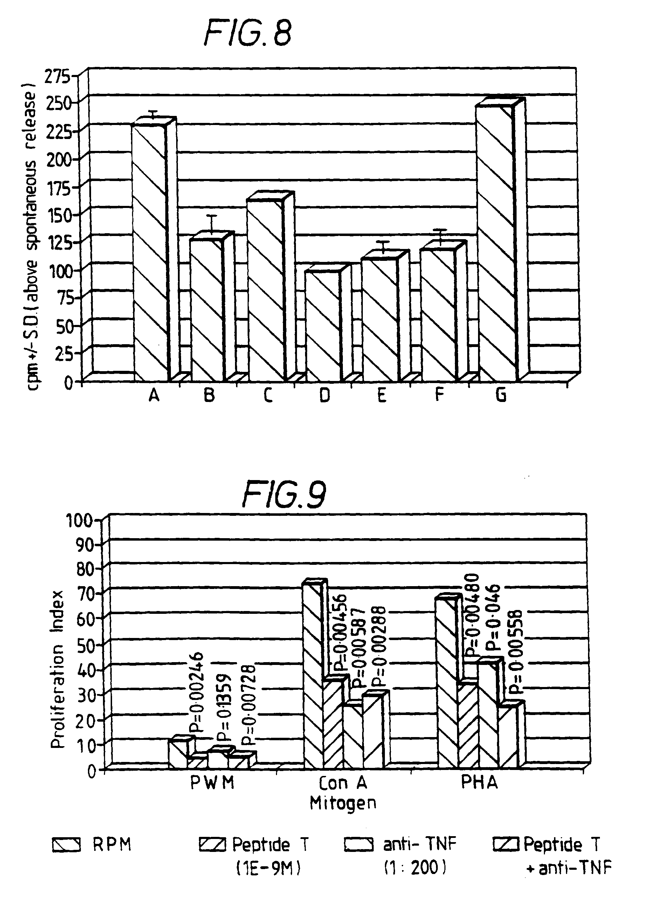

FIGURE 8, which also relates to Example 10, shows that the supernatant of macrophage cultures stimulated with LPS contain cytolytic substances capable of killing target K562 cells. Target cells are labelled with intracellular radioactivity that is released upon cell death. More radiolabel release (measured as counters per minute, cpm) indicates more killing. Int he absence of Peptide T (A) more killing occurred than in its presence (B-F). As the concentration of Peptide T was dropped below $1 \times 10^{-15}$M cytolytic activity the degree of cytotoxicity returned to control (A) levels. The Peptide T concentrations were as follows:

A: no Peptide T
B: Peptide T, $10^{-5}$M
C: Peptide T, $10^{-7}$M
D: Peptide T, $10^{-9}$M
E: Peptide T, $10^{-11}$M
F: Peptide T, $10^{-13}$M
G: Peptide T, $10^{-15}$M

FIGURE 9 is referred to in Example 11 and shows that Peptide T has a similar effect to anti-TNF in inhibiting the effects of TNF.

FIGURE 10 is referred to in Example 12 and shows that Peptide T decreases serum TNF levels in HIV patients.

FIGURE 11 is referred to in Example 14 and shows that Peptide T analogue 623 prolongs survival of sensitised mice administered a lethal dose of LPS.

FIGURE 12 is referred to in Example 15 and shows that Peptide T inhibits activation of human neutrophils by TNF.

FIGURE 13 is also referred to in Example 15 and shows that Peptide T analogue 505 inhibits TNF priming and activation of human neutrophils.

FIGURE 14 is again referred to in Example 15 and shows that Peptide T analogue 505 inhibits LPS activation of human neutrophils.

FIGURE 15 is referred to in Example 17 and shows that Peptide T reduces TNF-induced expression of tissue factor in HUVECs.

EXAMPLE 1 - LPS-INDUCED INFLAMMATION

Normal balb/c mice (female, 12 weeks) were administered LPS (250 µg, *E. coli* K-235, Sigma cat. no. L-2018) at time zero. One group of mice (50 animals) were then treated at 30 minutes intervals by i.p. injections of bovine serum albumin (BSA) (Sigma cat. no. 6793) dissolved in pyrogen-free, sterile, isotonic water (2.5 mg BSA per animal per injection, each injection containing 100 µl).

The second group of mice (50 animals) were treated at 30 minutes intervals by i.p. injections of Peptide T (Carlbiotech A/S batch 109401) dissolved in pyrogen-free, sterile, isotonic water (2.5 mg Peptide T per animal per injection, each injection 100 µl).

Glucose levels were determined on blood samples after 3 hours. Subjective parameters such as secretion from the eye, diarrhoea and general well being are indicated on a scale of (-) to (+++).

As can be seen from Table I, the difference in glucose levels between peptide T-treated animals and BSA-(placebo) treated animals is highly significant. The subjective parameters also strongly indicate a general higher index of well being for animals treated with Peptide T.

TABLE I

| | Diarrhoea | | Blood Glucose | | Secretion from eyes | | General well being | |
|---|---|---|---|---|---|---|---|---|
| | $t_0$ | | $t_0$ | $^3$3hrs | $t_0$ | | $t_0$ | |
| | $^t$3hrs | | | | $^t$3hrs | | $^t$3hrs | |
| Control (BSA) | - | +++ | 4.4=0.7 | 2.0=0.2 | - | +++ | - | +++ |
| Peptide T treated | - | + | 4.3=0.6 | 2.4=0.5 | - | + | - | + |
| t-test = 98 | | | | | | | | |
| p < 0.001 for peptide T treated group vs. control (BSA-treated group). | | | | | | | | |

EXAMPLE 2 - PEPTIDE T ANTAGONISES TNF

Stimulation of endothelial cells with TNF results in the induction of procoagulant activity through the expression of tissue factor activity and a concomitant reduction of the anticoagulant potential of the cells through reduced expression of thrombomodulin (Bevilacqua *et al,* Nawroth and Stern, *J. Exp. Med.* **163** 740 (1986)). Septic shock is frequently accompanied by disseminated intravascular coagulation (DIC, *Semeraro Acta Clinica Belgica* **31** 377 (1976)) and there is considerable evidence that tissue factor is an essential requirement for DIC to be induced by endotoxin (LPS). A monoclonal antibody against tissue factor has been shown to inhibit DIC induced by the administration of endotoxin (Warr *et al, Blood,* **75** 1481 (1990)).

Human umbilical vein endothelial cells were cultured in M199 medium supplemented with 10% foetal calf serum, endothelial cell growth factor, penicillin, streptomycin and L-glutamine in 96-well dishes using a modification of the procedure of Bevilaqua *et al, Proc. Natl. Acad. Sci. USA* **83** 4533 (1986)). TNF treatment of cultures (100 units.ml) was for 4 h at 37°C in the presence of growth medium, after which the cells were washed and fixed. Cultures were also treated with Peptide T + TNF or Peptide T alone. In these cultures Peptide T was at a concentration which ranged from 0.5 to 100 µg/ml. Tissue factor expression by the endothelial cells treated with TNF with or without peptide T was determined in an ELISA using a monoclonal antibody which neutralises the activity of human tissue factor (Carlbiochem A/S). Bound anti-tissue factor antibody was detected using peroxidase-conjugated rabbit anti-mouse Ig and the substrate ABST. Colour intensity was determined by the optical density of each well at 405nm.

The data obtained from triplicate cultures show that peptide T inhibited the expression of tissue factor on endothelial cells stimulated by 100 units/ml human recombinant TNF and are illustrated in Figure 1.

EXAMPLE 3 - PEPTIDE T INSTIGATES LPS-INDUCED MORTALITY

Mice bearing Meth A ascites tumours were given 100 µg LPS subcutaneously with either bovine serum albumin (BSA) or peptide T analogue (504 or 505, 1mg). The number of animals showing signs of LPS toxicity (diarrhoea, exudate from the eyes and general level of activity) in each group was recorded at 19, 24 and 41 hours after injection. At the completion of the experiment the number of surviving animals was recorded (see Table II below, and the survivors bled for later determination of TNF, IL-1, IL-6 and RNI levels.

The sequence of the most effective analogue (505 is:

H-D-Ser-Ser-D-Thr-Thr-D-Thr-Thr-Tyr-D-Thr-NH$_2$

and the sequence of the 504 is:

H-D-Ala-Ser-D-Thr-Thr-D-Thr-Asn-Tyr-D-Thr-NH$_2$.

TABLE II

| EFFECTS OF PEPTIDE T ANALOGUES ON SURVIVAL OF LPS-TREATED TUMOUR BEARING MICE | | | |
|---|---|---|---|
| Treatment | % mice surviving 41 hours | % mice with diarrhoea at 19 hrs | % mice with exudate at 19 hrs |
| LPS+BSA | 60 | 100 | 100 |
| LPS+505 | 100 | 40 | 20 |
| LPS+504 | 100 | 40 | 60 |

Examples 4 to 7 illustrate the effectiveness of peptides useful in the invention in relation to various symptoms associated with chronic inflammation.

EXAMPLE 4 - INHIBITION OF MITOGEN INDUCED PROLIFERATION OF PBMC

PBMC ($10^5$) were cultured in 200 µl RPMI containing 10% heat inactivated foetal bovine serum, 2 mM L-glutamine and gentamycin 100 µg/ml with mitogen and Peptide T added on the first day of culture. Growth was assessed in triplicate by $^3$H thymidine uptake following a 6 day incubation and expressed as a proliferation index (cpm in presence of mitogen ± Peptide T + cpm in absence of stimulation). A proliferation index >1.00 implies growth beyond that in media alone. At physiologically obtainable concentrations, Peptide T inhibits proliferation of normal (HIV seronegative) peripheral blood mononuclear cells (PBMC) without causing significant cytotoxicity as shown in Table III. This effect is long term in that Peptide T given on the first day of a six day culture will inhibit mitogen induced proliferation.

TABLE III

| Proliferation Index ± SD[1] | | | | |
|---|---|---|---|---|
| Peptide T[2] | Media[3] | PWM[4] | ConA[4] | PHA[4] |
| 0 | 1.00 | 7.47±0.50 | 4.55±0.66 | 317.0±41.0 |
| $10^{-5}$ | 1.14±0.15 | ND[5] | 2.72±1.20 | 149.0±38.4 |
| $10^{-7}$ | 0.73±0.16 | 4.47±0.36 | 3.19±0.06 | 123.6±18.1 |
| $10^{-9}$ | 2.17±1.10 | 4.69±0.42 | 1.98±0.04 | 78.6±23.1 |
| $10^{-12}$ | 1.52±0.15 | 4.15±0.23 | 1.69±0.48 | 103.8±22.8 |

1. SD - standard deviation
2. Concentration of peptide T in mol/l
3. Growth in absence of mitogens
4. Pokeweed mitogen (PWM), Concanavalin A (Con A) and phytohemagglutinin (PHA) were used at 25 µg/ml
5. ND - not done

## EXAMPLE 5 - INHIBITION OF MLR

A two way MLR was performed using $10^5$ allogeneic PBMC (cpm of A = 142 ± 86, B = 148 ± 84) cocultured in 200 μl of media as described in Table III.

Peptide T given on the first day of an MLR will inhibit allogeneic induced proliferation of PBMC (Table IV).

TABLE IV

| Inhibition of MLR | |
|---|---|
| Peptide T[1] | cpm ± SD[2] |
| 0 | 1365 ± 335 |
| $10^{-5}$ | 649 ± 263 |
| $10^{-7}$ | 534 ± 108 |
| $10^{-0}$ | 764 ± 77 |
| $10^{-11}$ | 681 ± 222 |
| $10^{-13}$ | 235 ± 4 |
| $10^{-15}$ | 371 ± 117 |
| $10^{-17}$ | 672 ± 261 |

1. Concentration of peptide T in mol/l
2. Counts per minute ± standard deviation

## EXAMPLE 6 - EFFECT OF PEPTIDE T ON ACTIVITY OF p56[lck] IN PBL BLASTS

PBL (peripheral blood leukocyte) blasts were prepared by incubating PBMC at 37°C with PHA (10 μg/ml) in RPMI + 10% FBS for 48 hours followed by addition of interleukin-2 and incubation for a subsequent 96 hours. Cells were washed free of IL-2 and incubated for 24 hours then washed free of FBS and incubated for 24 hours. PBL blasts remained either stimulated or were treated with OKT4A (anti-CD4) at 10 μg/ml for 1 hour followed by rat anti-mouse Ig at 5 μg/ml for 5 minutes to enhance lck activity. PBL blasts were pretreated with Peptide T ($10^{-9}$ M) for 2 hours, then washed extensively before anti-CD4 stimulation. Following immunoprecipitation with anti-lck-Protein A conjugates and incubation (37°C, 10 minutes) with $^{32}$P-ATP, p56[lck] autophosphorylation was determined by autoradiography and used as a measure of p56[lck] activity.

Peptide T will inhibit the PHA-induced activation and anti-CD4 dependent enhancement of p56[lck] activity as we have determined (Table V) by an lck specific tyrosine kinase assay. p56[lck] is a CD4 associated tyrosine kinase involved in T cell activation and transmembrane signalling (Janeway C.A. *Ann. Rev. Immunol.* **10** 645-674 (1992)). In lieu of a T cell receptor associated signal, cross-linking of CD4 by monoclonal anti-CD4 inhibits T cell activation (Burgess *et al. Eur. J. Immunol.* **21** 1663-1668 (1991)), possibly by activating p56[lck] including autophosphoryllation of the inhibitory Tyr 505 (Abraham *et al, Nature* **350** 62-66 (1991); Abraham *et al, Cancer Invest.* 9 455-463 (1991)). Peptide T is able to inhibit p56[lck] activation (Table V) thus achieving the same result as anti-CD4 (inhibition of T cell activation) although by a different mechanism.

TABLE V

| EFFECT OF PEPTIDE T ON ACTIVITY OF p56[LCK] IN PB< BLASTS | | | |
|---|---|---|---|
| Unstimulated | | Anti-CD4 | |
| -P.T.[1] | +P.T. | -P.T. | +P.T. |
| +++[2] | + | ++++ | ++ |

1 - P.T. means Peptide T
2 - ++++ highly active
+ slightly active

It is known that anti-CD4 antibodies will inhibit mitogen-induced as well as and antigen-induced *in vitro* proliferation of PBMC, lymphokine release, T helper function and MLR (Bank *et al, J. Exp. Med.* **162** 1294-1303 (1985)). There are two possible mechanisms of anti-CD4 inhibition of T cell function.

1) Anti-CD4 antibodies cause steric hindrance of the CD4/MHC II interaction that occurs during T cell activation. Peptide T would probably not share this effect since it is derived from a sequence of gp120 known to bind to CD4 and the binding site of gp120 on the CD4 molecule is distant from the MHC II binding site (Fleury *et al* (1991)).

2) Cross-linking of CD4 by multivalent anti-CD4 antibodies, in lieu of TcR/CD3 stimulation sends a negative signal to the T cell (Bank *et al, J. Exp. Med.* **162** 1294-1303 (1985)). Peptide T is monovalent, hence cannot cross-link CD4 receptors; therefore, its mechanism of inhibition of T cell activation must be distinct from that of anti-CD4. Treatment of inflammatory conditions with mouse anti-CD4 has resulted in a number of undesirable side effects including urticaria, chills, fever and tremors that occasionally require medical intervention. In addition, following administration of anti-CD4 there has been reported a reduction in CD4 cell numbers, a reduction in proliferation to mitogens and antigens and a reduction in delayed hypersensitivity reactions, a measure of cell mediated immune function (Horneff *et al, Cytokine* 3 266-267 (1991); Horneff *et al, Arth. Rheum.* **34** 129-140 (1991); Wofsey *et al. Semin. Immuno.* **2** 419-425 (1990)). Administration of anti-CD4 concurrently with antigen can inhibit both primary and secondary immune responses (Waldor N.K. *Immunol. Ser.* **45** 573-586 (1990)). In addition, the use of xenogeneic (non-human) proteins as therapeutic agents is limited due to the potential formation of antibodies directed against the foreign protein which can mediate shock and type III hypersensitivity reactions upon repeated administration.

## EXAMPLE 7 - EFFECT OF PEPTIDE T ON ANTIGEN INDUCED PBMC PROLIFERATION

PBMC were incubated as described in relation to Table III in Example 4 in the presence of live, attenuated measles, mumps and rubella vaccine (Merck Sharp and Dohme, Canada) at a final dilution of 1:20. Peptide T was added either on day 1 of incubation or on a daily basis. Results are expressed as a proliferation index ± standard deviation.

TABLE VI

| EFFECT OF PEPTIDE T ON ANTIGEN INDUCED PMBC PROLIFERATION | | |
|---|---|---|
| Conc. Peptide T (Mol/L) | Proliferation Index ± S.D. | |
|  | Day 1 | Daily |
| 0 | 3.26 ± 0.55 | |
| $10^{-7}$ | 4.02 ± 0.78 | 1.83 ± 0.36 |
| $10^{-9}$ | 1.91 ± 0.46 | 0.98 ± 0.16 |
| $10^{-11}$ | 5.35 ± 1.46 | 1.56 ± 0.14 |
| $10^{-13}$ | 5.46 ± 1.37 | 1.38 ± 0.51 |
| $10^{-15}$ | 5.56 ± 0.60 | 1.56 ± 0.61 |
| $10^{-17}$ | 3.99 ± 0.18 | 1.60 ± 0.60 |
| $10^{-19}$ | 6.15 ± 2.59 | 2.27 ± 1.09 |

Although Peptide T can inhibit antigen-induced proliferation of PBMC when administered *in vitro* on a daily basis, we do not believe this will have any significant effect on the normal immune response when administered *in vivo* (as demonstrated by Goodwin *et al* V. International Conf. Aids. Montreal. July 1989 Abstract #WBP286). There are two possible explanations for this that are not mutually exclusive.

1. Macrophages may be more sensitive to Peptide T than are T cells, since T cells receive many more potential activating signals than the CD4/MHC II interaction. Thus macrophages may be down regulated by Peptide T administration on the first day of *in vitro* culture inhibiting their ability to present mitogens and antigens to T cells. B cells can act as APC in place of macrophages when presenting antigen, but not when presenting mitogens which are antigen non-specific (explaining the results of Table III). Resting B cells are resistant to Peptide T as they are CD4 negative. In the presence of a single dose of Peptide T, they could present antigen to T cells allowing proliferation of PBMC in response to antigen. When Peptide T is given on a daily basis in cultured T cells, which may be less sensitive to the effects of Peptide T, then macrophages may be inhibited. This would explain the results shown in Table 4. Daily administration of Peptide T *in vivo* may not function as it does on a daily basis *in vitro,* since the half life of Peptide T *in vivo* is shorter than *in vitro. In vitro* serum supplements are heat inactivated to destroy proteolytic enzymes which actively degrade Peptide T *in vivo.* Thus daily administration of Peptide T *in vivo* would result in the inhibition of macrophage activity, but not T cell activity and would allow the continued expression of antigen driven immune responses. This would explain the efficacy of Peptide T in chronic inflammatory conditions such as rheumatoid arthritis (see below) where the characteristic inflammatory cell is the macrophage. Acute inflammation is characterised by neutrophil (CD4 negative) infiltrates and would not be affected by Peptide T administration. The requirement for antigen presentation (phagocytosis, degradation and presentation) in an antigen driven immune response results in a delay in T cell signalling. Hence Peptide T present on the first day of *in vitro* culture will not affect T cell signalling on subsequent days. Mitogen stimulation of PBMC proliferation (Table III) is an immediate event not requiring antigen processing and may be inhibited on a long term basis by concurrent Peptide T administration. Peptide T needs to be present concurrently with an activating signal to manifest its immunodepressive effects. During chronic inflammation or immune activation, all responding cells will be receiving proliferation signals all the time. Daily Peptide T administration *in vivo* will inhibit chronic immune activation. Since the half life of Peptide T is <1 hour (Ruff *et al, Prog. Neuro-Psychopharmacol & Biol Psychiat* Vol. 15, pp 791-801 (1991)), those proliferation signals active during the first hour after Peptide T administration will be inhibited. since all cells responding to chronic activation signals respond all the time, they will be down regulated during this 1 hour period and remain down regulated on a long term basis. Due to the discrete timing of antigen presentation and the fact that not all epitopes of the antigen are presented at the same time (therefore not all T cells capable of responding are activated at the same time), there are 23 hours before the next injection of Peptide T in which to mount antigen specific immune responses. Although some antigen specific immune responses may be inhibited in the 60 minutes post Peptide T administration, the majority which are happening at discrete times, will be allowed to proceed.

In this fashion, Peptide T is able to inhibit chronic immune activation and inflammation while allowing the expression of antigen specific immune responses.

Peptide T appears to act as an anti-inflammatory agent. Peptide T would therefore be a novel non-steroidal anti-inflammatory drug (NSAID). Tradional NSAIDs, such as aspirin and ibuprofen, have two potential mechanisms of action: inhibition of cyclooxygenase reducing prostaglandin production and inhibition of neutrophil function (Altman R.D. *Arth. Rheum.* **19** (Suppl. 2) 1-5 (1990); Vane *et al, Postgrad. Med J.* **66** (Suppl. 4) S2-S17 (1990)). It had been reported that inhibition of cyclooxygenase will enhance (rather than suppress) mitogen driven PBMC proliferation (Vane *et al., Int. J. Immunopharmacol.* **5** 107-114 (1983)) and neutrophils (CD4 negative) would be resistant to Peptide T; therefore, Peptide T may have a unique mechanism of anti-inflammatory action.

The anti-inflammatory azathioprine inhibits lymphocyte proliferation by inhibiting DNA and RNA synthesis. The proliferation of both B and T lymphocytes are inhibited by the action of azathiporine (Briggs J.D. *Immunol. Lett.* **29** 89-94 (1991)). Since B cells are CD4 negative and can function as APC in the presence of Peptide T (see above), Peptide T must have a mechanism of action distinct from that of azathiporine.

Cyclosporine is an anti-inflammatory drug that inhibits lymphocyte proliferation by inhibiting IL-2 and IFN γ gene transcription by binding to and inactivating cyclophilin, an isomerase required for signal transduction (Halloran *et al, Clin. Biochem.* **24** 3-7 (1991)). While Peptide T may also affect signal transduction (Table 3), it does not have the severe side effects associated with cyclosporine therapy. Cyclosporine induced side effects include renal tubular atrophy, nephrotoxicity, hypertension, hirsutism, gingival hypertrophy, tremors, convulsions and paresthesia (Briggs J.D. *Immunol. Lett.* **29** 89-94 (1991)). In the experience of the present invention, no significant side effects have been attributable to Peptide T therapy.

EXAMPLE 8 - PEPTIDE T INHIBITS MITOGEN-INDUCED LYMPHOPROLIFERATION

Concanavalin A (Con A), pokeweed mitogen, (PWM) and phytohaemagglutin-P (PHA-P) were obtained from Sigma Chemical Co. (St. Louis, MO, USA). Peptide T was synthesised by Carlbiotech Ltd (Copenhagen, Denmark) and supplied as the chloride salt at 8.5 mg/ml in 0.9% benzyl alcohol as a preservative and was stored at 4°C. Peptide T was diluted to working solutions in culture medium (RPMI). Dilute solutions of Peptide T were prepared fresh from stock Peptide T for each experiment. $^3$H-thymidine (5 Ci/mmol) was obtained from Amersham.

*Isolation of PBMC*: Whole blood from healthy, adult volunteers was drawn into heparin and peripheral blood mononuclear cells (PBMC) isolated by density gradient centrifugation on HISTOPAQUE™ (Sigma Chemical Co). PBMC were removed from the interface, washed three times in RPMI containing gentamycin (100µg/ml) and resuspended to

$1x10^6$ PBMC/ml in RPMI containing 10% foetal bovine serum and gentamycin. $10^5$ PBMC were cultured in the presence of 100µl of mitogen suspension in 96 well plates. The final concentration of mitogen was: PWM 25µg/ml; PHA 12.5µg/ml and Con A 6.25µg/ml. 10µl of Peptide T working solution (or RPMI as negative control) was added and cell cultures incubated for a total of 5 days in a humid atmosphere, 5% $CO_2$, 37°C. Cultures were then pulsed with $^3$H-thymidine (in RPMI) 18-24 hours prior to harvesting. The amount of incorporated $^3$H-thymidine was determined using a BECKMAN™ LS8000 liquid scintillation counter and proliferation expressed as either counts per minute (cpm) or as a proliferation index (PI).

$$PI = \frac{\text{cpm of (PBMC+mitogen)}}{\text{cpm of (PBMC+media)}}$$

A PI of 2 indicates that cells have grown to twice their resting (unstimulated) number.

All experiments were performed in triplicate. Results reported are characteristic of replicated experiments.

*Production of Tumour Necrosis Factor* α *(TNFα)*: $5x10^6$ PBMC/ml were incubated in the presence of Con A (6.25µg/ml) for 48 hours in a humid atmosphere, 5% $CO_2$, 37°C. Peptide T ($10^{-9}$M) was added at t=0 and 24 hours. At the end of incubation, SEPHADEX™ G25 (Pharmacia) was added (10mg/ml) and incubated for 10 minutes to remove mitogen. PBMC and SEPHADEX were pelleted by centrifugation and the supernatant tested for levels of TNFα using a TNFα radioimmune assay kit from Amersham. Samples were analysed in duplicate.

*Statistical Analyses*: Data were analysed by a two sample ANOVA test using 95% confidence limits such that a value of $p \leq 0.05$ was considered a significant difference.

Table VII

| $1x10^5$ PBMC were cultured in the presence of mitogens with Peptide T ($1x10^{-9}$M) added concurrently with mitogens (Day 1) or on subsequent days as indicated. Cells were cultured for a total of 6 days. Results are expressed as proliferation index ± standard deviation. | | | | |
|---|---|---|---|---|
| | | PWM | Con A | PHA |
| no Peptide T | | $4.29 \pm 0.25$ | $21.0 \pm 5.7$ | $197 \pm 3$ |
| Peptide T: | Day 1 | $3.13 \pm 0.21$ | $6.2 \pm 0.5$ | $148 \pm 13$ |
| | Day 2 | $3.20 \pm 0.20$ | $6.0 \pm 1.1$ | $101 \pm 7$ |
| | Day 3 | $2.65 \pm 0.17$ | $9.9 \pm 0.1$ | $148 \pm 7$ |
| | Day 4 | $3.22 \pm 0.28$ | $9.5 \pm 1.6$ | $131 \pm 10$ |

**Results**

When added on the first day of culture, Peptide T was able to inhibit lymphoproliferation in response to PWM (Figure 2a), Con A (Figure 2b) and PHA (Figure 2c). Peptide T was able to inhibit PHA stimulated PBMC cultures at a concentration of $1x10^{-13}$M. Cultures stimulated with Con A or PWM were inhibited by as little as $1x10^{-15}$M peptide T. Peptide T reaches peak plasma concentrations of $5.5x10^{-8}$M following administration *in vivo*; therefore, Peptide T mediated immunomodulation would be active at concentrations of the drug readily attainable in plasma and CSF. The culture medium, in which Peptide T was diluted, had no effect on lymphoproliferation. At the most effective concentration of Peptide T used, lymphoproliferation in response to PHA was inhibited 26%, PWM 38 and Con A 36%.

Inhibition of lymphoproliferation could be a result of cytotoxicity, a delay in initiation of proliferation, a reduction in growth rate or any combination of the above.

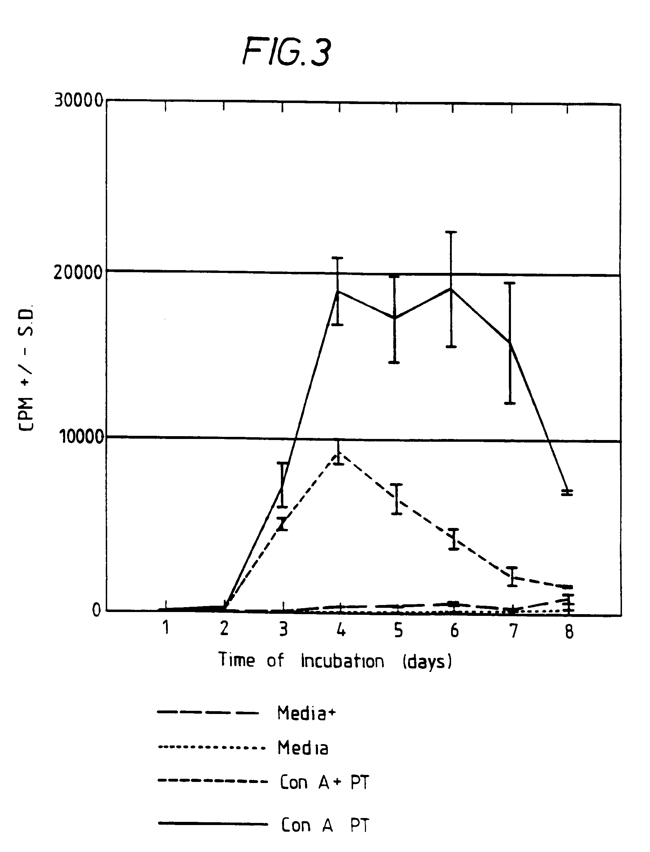

When the Con A-induced proliferation of PBMC was measured daily, during the growth period (day 2-4) lymphocyte proliferation in the presence of Peptide T paralleled, but at a reduced race, growth in the absence of the drug (Figure 3). In the absence of mitogen, Peptide T had negligible effects on PBMC. Therefore, Peptide T was not cytotoxic for responding lymphocytes nor did it delay the onset of proliferation. Peptide T allowed cells to grow, but at a reduced rate.

Peptide T was able to inhibit mitogen induced lymphoproliferation when added to PBMC after they had been exposed to mitogens (Table VII). This effect was present even when Peptide T was added three days after exposure of PBMC to mitogen. Peptide T mediated inhibition of lymphoproliferation was most pronounced when added to cultures that had been stimulated with Con A.

In addition to inhibition of lymphoproliferation, the presence of Peptide T reduced the amount of TNFα secreted into the supernatant (SN) of a 48 hour Con A stimulated culture of PBMC (Table VIII). Neither Peptide T nor RPMI had any reactivity in the RIA for TNFα. In the absence of Con A, no TNFα was detected in the culture supernatant.

Table VIII

| PBMC (5x10$^6$ PBMC/ml) were cultured for 48 hours in the presence or absence of Con A (6.25μg/ml) = Peptide T (10$^{-9}$M, added concurrently with mitogen). Levels of TNFα in culture supernatants were determined as described. | |
| --- | --- |
| | TNFα (fmol/100ul) |
| RPMI+Peptide T | <1 |
| PBMC+RPMI+Peptide T | <1 |
| PBMC+Con A | 69.0 ± 6.9 |
| PBMC+Con A+Peptide T | 47.0 ± 0.1 |

EXAMPLE 9 - PEPTIDE T INHIBITS NON-MITOGEN-INDUCED LYMPHOPROLIFERATION

In contrast to mitogens which induce lymphocyte proliferation signals by ligating MO/MAC and T cells via carbohydrate moieties on a variety of cell surface receptors, non-mitogen stimulated lymphoproliferation mediated by recall antigens, superantigens, antibodies to CD3 (anti-CD3) and allogeneic MHC molecules interact solely with the T cell antigen receptor/CD3 complex (TcR/CD3). In this respect, non-mitogen stimuli are more specific than mitogens.

Only those lymphocytes with TcR complementary to processed antigen epitopes will respond to recall antigens. Typically this represents a small fraction of the total lymphocyte pool. Reactions to allogeneic MHC, characterised by the mixed lymphocyte reaction (MLR) is mediated by recognition of foreign MHC Class II molecules by the TCR of responding cells (*Adv. Immunol.* **31** 271 (1981)). Approximately 10% of T cells are alloreactive (*Lancet* **339** 824 (1992)). Superantigens such as *Staphylococcal* enterotoxins A and B (SEA and SEB) and toxic shock syndrome toxin-1 (TSST-1) stimulate growth of approximately 20% of the T cell pool (*Sci. Am.* **266** 92 (1992)) by interaction with defined V$_\beta$ segments of the TcR (*Science* **244** 811 (1989)). Anti-CD3 is able to stimulate the growth of 100% of T cells independent of accessory cell function by ligating TcR/CD3 complexes directly.

Since mitogens represent an artificial, non-specific form of lymphocyte activation, the effect of Peptide T on lymphoproliferation in response to recall antigens, anti-CD3, superantigens and MLR was assessed. In addition, T cell effector functions and the growth of spontaneously proliferating malignant cell lines were examined in the presence of Peptide T.

Unless otherwise stated, experimental conditions were generally as described in Example 9.

*Peptide T does not inhibit non-mitogen induced lymphoproliferation*

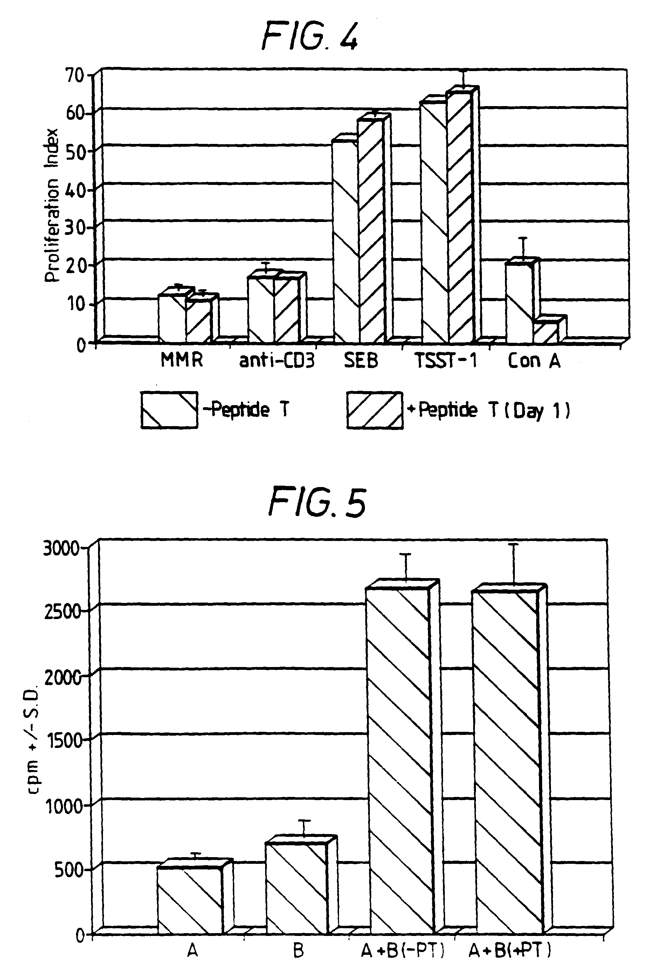

When added on the first day of a six day culture, Peptide T was able to inhibit Con A-induced lymphoproliferation, but had negligible effects on lymphocytes responding to recall antigen (measles, mumps, rubella vaccine, MMR), antibodies to CD3 or the superantigens SEB and TSST-1 (Figure 4). Similarly, Peptide T did not affect the growth of lymphocytes in a mixed lymphocyte reaction (Figure 5).

Molt-4 is a human T lymphoma cell line, p56$^{lck}$ negative, and Jurkat is a human T cell line, p56$^{lck}$ positive, that will grow spontaneously in the absence of exogenous stimuli. As the results of Table IX demonstrate, Peptide T did not significantly affect the growth of either Molt-4 or Jurkat cell lines.

Table IX

| Effect of Peptide T on Growth of Cell Lines | | |
|---|---|---|
| Two spontaneouly growing human malignant T cell lines (Jurkat and Molt-4) were grown in the absence (Media-P.T.) or presence (Media+P.T.) of Peptide T added on the first day of culture. Growth was determined by measurement of incorporated $^3$H-thymidine; counters per minute (cpm) were not converted into a proliferation index. A higher cpm represents greater cell growth. | | |
| Jurkat | Media - P.T. (CPM±S.D.) | Media + P.T. (CPM±S.D.) |
| Day 1 | 7445 ± 261 | 8345 ± 131 |
| Day 2 | 14728 ± 1349 | 17441 ± 1360 |
| Day 3 | 41203 ± 6127 | 40002 ± 1572 |
| Day 4 | 50930 ± 1030 | 49852 ± 1011 |
| Day 5 | 80046 ± 1072 | 79748 ± 8474 |
| Day 6 | 109201 ± 8805 | 114967 ± 7342 |
| Day 8 | 113667 ± 7266 | 109144 ± 14052 |
| | | |
| Molt - 4 | Media - P.T. (CPM±S.D.) | Media + P.T. (CPM±S.D.) |
| Day 1 | 6182 ± 1296 | 6639 ± 726 |
| Day 2 | 13111 ± 370 | 15001 ± 1509 |
| Day 3 | 29175 ± 5594 | 32401 ± 850 |
| Day 4 | 39920 ± 3407 | 39839 ± 10740 |
| Day 5 | 54686 ± 1472 | 50269 ± 5436 |
| Day 6 | 56824 ± 3580 | 57570 ± 4968 |
| Day 8 | 10835 ± 264 | 19510 ± 4217 |

*Peptide T does nor inhibit PBMC mediated killing of Molt-4 cells*

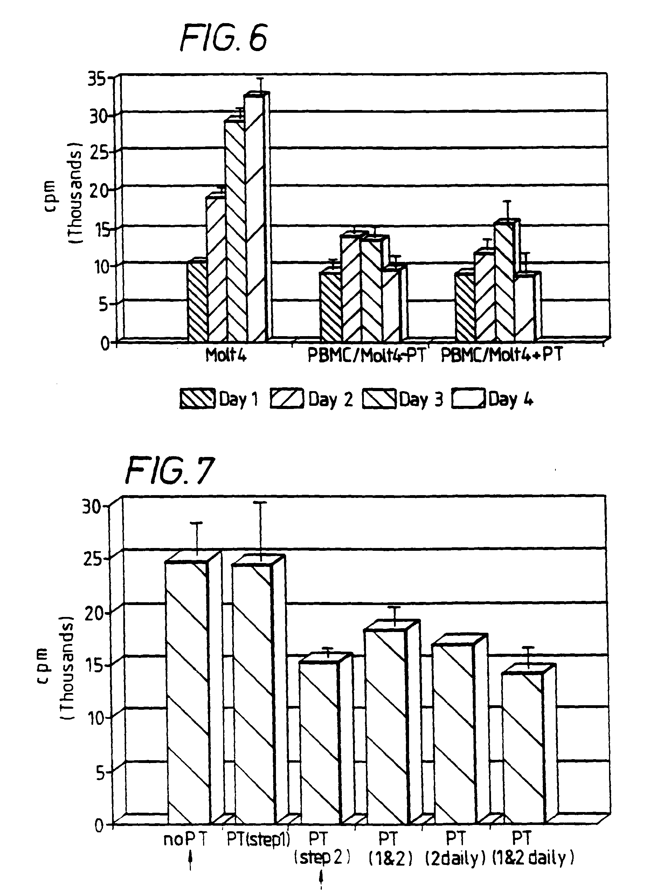

When PBMC are mixed with allogeneic Molt-4 cells the growth of Molt-4 is inhibited (Figure 6). Alloreactive PBMC recognise Molt-4 as foreign and generate cytotoxic effector cells that kill Molt-4. Molt-4 is not a cytotoxic cell, line and can not generate alloreactive effecter cells. When Peptide T is added to this reaction; PBMC-mediated inhibition of Molt-4 proliferation remains unaffected (Figure 6) suggesting that Peptide T does not affect the generation of cytotoxic effector functions. It is not possible to distinguish growth of alloreactive T cells from the growth of Molt-4 survivors since neither the growth of alloreactive T cells (Figure 5) nor the growth of Molt-4 (Table IX) would be affected by Peptide T.

EXAMPLE 10 - PEPTIDE T's EFFECT ON MACROPHAGE AND TNF FUNCTION

Cells of the monocyte/macrophage lineage (MO/MAC) are long lived, non-proliferating cells that characterise chronic inflammatory reactions. MO/MAC function as non-specific phagocytes, antigen presenting cells (initiating antigen specific immune responses), as immunoregulatory cells and cytotoxic effector cells. MO/MAC generate a variety of intracellular and extracelluar cytotoxic effector molecules including reactive oxygen intermediates, lysosomal enzymes and tumour necrosis factor alpha (TNF).
MO/MAC have been implicated in the pathogenesis of HIV disease. All MO/MAC express CD4 (*J. Immunol. Meth.* **135** 59 (1990)) and are therefore capable of being infected with HIV. It has even been suggested that MO/MAC maybe the first cell to be infected with HIV during the infection process (*Annal. N.Y. Acad. Sci.* **616** 1 (1990)). MO/MAC are not as susceptible to the cytopathic effects of HIV as are CD4 positive T cells (*TIPS* **12** 28 (1991)) and may be responsible for the establishment of a persistent or latent HIV infection. MO/MAC may be responsible for transmission of HIV into the CNS where it comprises the primary cell type infected with HIV (*Compr. Ther.* **17** 57 (1991)). In the CNS, MO/MAC may mediate neurological damage by releasing neurotoxic gp120 (*Science* **248** 364 (1990)), *tat* (*J. Virol.* **65** 961 (1991)) or MO/MAC derived neurotoxins (*Science* **250** 1593 (1990)). It is interesting to note that activated MO/MAC have also been implicated in the pathogenesis of other neurological disorders such as multiple sclerosis (*Acta. Neuropathol.* **80** 208 (1990); *Pathol. Immunopathol. Res.* **6** 241 (1987); *Ann. Rev. Immunol.* **10** 153 (1992)) and Alzheimer's disease (*Eur. Neurol.* **28** 30 (1988)).

HIV-infected MO/MAC exhibit accessory cell dysfunction (*Clin. Immunol. Immunopathol.* **59** 436 (1991); *J. Immunol.* **146** 2297 (1991)). In addition, HIV induces MO/MAC activation as indicated by enhanced levels of neopterin, a marker of T cell dependent MO/MAC activation, and enhanced levels of monokines (TNF, IL-1 and IL-6).

*In vitro,* MO/MAC may be activated by IFN-γ and lipopolysaccharide (LPS) which initiate at leat four distinct intracellular signals including a calcium flux (*Immunol. Today* **10** 33 (1989)). The production of TNF may be initiated by IFN activation of MO/MAC but requires LPS to trigger its release (*J. Exp. Med.* **175** 405 (1992)).

A role for TNF in HIV disease has been suggested based on the observations that macrophages from AIDS patients release greater amounts of TNF than those from normal controls (*Am. Rev. Respir. Dis.* **144** 195 (1991)) and increased levels of TNF have been associated with progression from latent HIV infection to AIDS (*Am. J. Med.* **85** 289 (1988)). Potential effects of TNF contributing to HIV pathogenesis include TNF mediated cachexia (*New Eng. J. Med* **327** 329 (1992); *J. Nutr*. **122** 749 (1992)), autocrine/paracrine enhancement of HIV replication (*Immunol. Today* **11** 176 (1990)), hyperactivation of the immune system (*Biotherapy* 3 127 (1991)), inhibition of zidovudine (AZT), ddI and ddC (*VIII Int'l Conf. AIDS* Abst.#PoA 2326 (1992); *J. Intern*. *Med*. **228** 549 (1990)), expansion of autoreactive CD4 specific CTL (*VIII Int'l Conf. AIDS* Abst.#PoA 2365 (1992)), damage to myelin and oligodendrocytes (*AIDS* 5 1405 (1991)), cytopenia (*Am. Rev. Respir. Dis.* **144** 195 (1991)) and apoptosis (*Immunol. Ser.* **56** 315 (1992)).

TNF has also been implicated in the pathogenesis of multiple sclerosis. TNF is cytotoxic for myelin producing oligodendrocytes (Ann. *Rev. Immunol.* **10** 153 (1992)) and can cause myelin damage directly by inducing blebbing and swelling of the myelin sheath (*Annal. Neurol.* **23** 339 (1988)).

**Methods**

*Macrophage Cytotoxicity Assay*

The MO/MAC cytotoxicity assay is a two step assay. After isolation of adherent cells (mainly MO/MAC) from PBMC, MO/MAC are activated by lymphokines (including IFN gamma and LPS. Subsequent to washing which removes activators, $^3$H-thymidine labelled K562 target cells were added to activated MO/MAC and incubated for 3 days. The degree of cytotoxicity was proportional to the amount of radioactive label released into the culture supernatant, is shown in the following scheme:

```
                          PBMC
                            |
        Adherent Cells (1 hours, 37°C on plastic)
                            |
            Step 1: Activation (8 hour): cytokines
                                          LPS (1ug/ml)
                            |
             Step 2: Effector (3 days): ³H-T K562
                            |
                      Release  of ³H-T
```

*MO/MAC Supernatant Cyrotoxicity*

Supernatant (SN) of activated MO/MAC were collected from monolayers of PBMC derived adherent cells that had been exposed to LPS for 23 hours. This SN was tested for cytolytic activity against $^3$H-thymidine labelled K562 cells in a 48 hour, 37°C cytotoxicity assay. SN were stored at 4°C overnight or stored frozen at -20°C for longer periods before using.

**Results**

*Peptide T inhibits MO/MAC mediated cytotoxicity*

The presence of Peptide T at different stages of the cytotoxicity assay demonstrated that while Peptide T did not affect the ability of resting MO/MAC to be activated, it was able to inhibit the cytotoxicity of activated MO/MAC (Figure 7). Whether Peptide T was added concurrently with target cells or every day of the culture did not affect the drug's activity.

*Peptide T treatment reduces neopterin levels but Peptide T-dependent decreases in serum neopterin were not associated with decreases in serum IL-1.*

When sera from patients with Peptide T dependent decreases in elevated neopterin levels were tested for IL-1, no correlation between the two could be demonstrated (Table X). However, neopterin levels were elevated prior to Peptide T administration whereas serum IL-1 levels were normal.

Table X

| Serum IL-1 and Neopterin Concentration | | | | |
|---|---|---|---|---|
| Serum was collected from patients with HIV disease and stored at -20°C. Neopterin and IL-1 concentrations were determined by RIA. Pre- and post-Peptide T values of IL-1 and neopterin were determined for the same serum samples. | | | | |
| PATIENT | [IL-1] (fmol/100μl) | | [Neopterin] (nM) | |
|  | pre-Peptide T | post-Peptide T | Pre-Peptide T | post-Peptide T |
| norm. | $4 \pm 0.03$ | N/A | ND | ND |
| BL | $4.1 \pm 0.03$ | $4.7 \pm 0.05$ | ND | ND |
| CJ | $3.7 \pm 0.03$ | $4.2 \pm 0.02$ | ND | ND |
| TS | $4.2 \pm 0.14$ | $4.2 \pm 0.26$ | ND | ND |
| LB | $3.9 \pm 0.13$ | $4.35 \pm 0.003$ | ND | ND |
| BG | $4.35 \pm 0.06$ | $4.6 \pm 0.02$ | $52.0 \pm 4.0$ | $16.85 \pm 0.05$ |
| BC | $3.5 \pm 0.14$ | $3.8 \pm 0.12$ | $47.3 \pm 0.8$ | $32.5 \pm 3.5$ |
| JM | $5.23 \pm 0.17$ | $4.2 \pm 0.07$ | $27.8 \pm 0.8$ | $19.75 \pm 0.25$ |
| RH | $4.1 \pm 0.01$ | $4.6 \pm 0.11$ | $13.15 \pm 2.85$ | $8.9 \pm 0.6$ |

*Peptide T inhibits cytotoxicity of MO/MAC culture supernatants.*

The ability of MO/MAC culture SN to lyse target K562 cells was determined in the presence of Peptide T. MO/MAC culture SN was used at a final dilution of 1 : 6 in the culture well. As shown in Figure 8, Peptide T was able to block MO/MAC SN mediated lysis of K562 even when added at $1 \times 10^{-13}$M.

EXAMPLE 11 - PEPTIDE T HAS A SIMILAR EFFECT TO ANTI-TINE IN INHIBITING THE EFFECTS OF TNF

$10^5$ PBMC were cultured in the presence of PWM, Con A or PHA (see Example 8). Peptide T ($10^{-9}$M), anti-TNF MoAb #47 at a 1:200 dilution (antiTNF) or a combination of them both were added to cultures concurrently with mitogens. Following a five day incubation, cultures were pulsed with $^3$H-thymidine 18-24 hours prior to harvesting. Incorporated $^3$H-thymidine was converted to a proliferation index (see Example 17). Data were analysed by two sample ANOVA using 95% confidence limits.

EXAMPLE 12 - PEPTIDE T DECREASES SERUM TNF LEVELS IN HIV PATIENTS

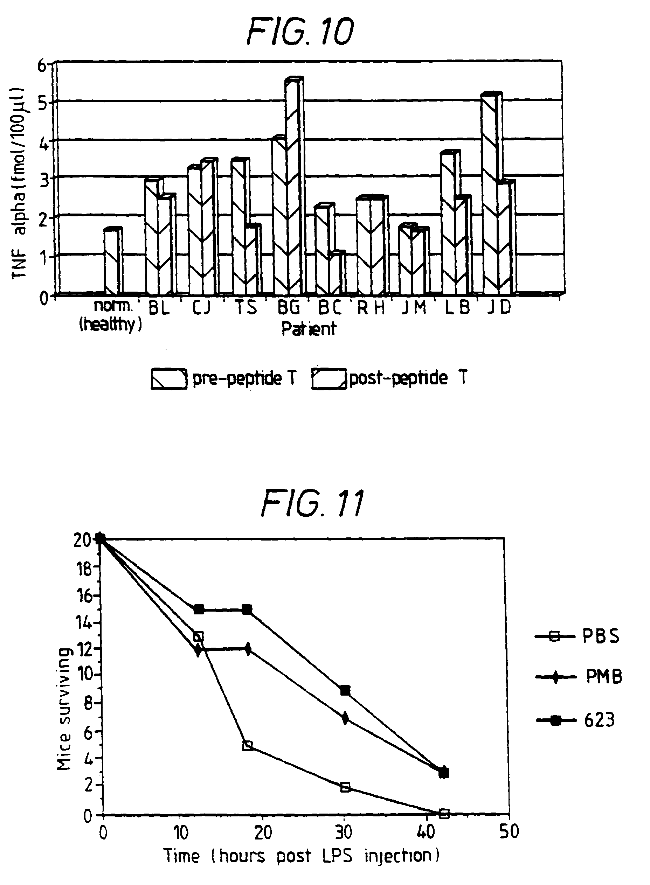

Serum levels of TNF were determined in patients with stage IV HIV disease pre- and post-Peptide T treatment. Patients had been receiving drug from 2 weeks to 11 months. Results are shown in Figure 10. The increase in serum TNF levels in patient BG was associated with the development of an opportunistic infection.

EXAMPLE 13 - PEPTIDE T PROLONGS SURVIVAL OF D- GALACTOSAMINE-SENSITIZED MICE IN A SEPTIC SHOCK MODEL

Fifty BALB/c /Swiss (1st cross) female mice approximately 8 weeks old were split into five groups and dosed as follows:

1. 16mg D-galactosamine + 20µg TNF + H$_2$O
2. 16mg D-galactosamine + 20µg TNF + MAb 47
3. 16mg D-galactosamine + 20µg TNF + 100µg Peptide T
4. 16mg D-galactosamine + 20µg TNF + 10µg Peptide T
5. 16mg D-galactosamine + 20µg TNF + 1µg Peptide T

D-galactosamine sensitizes the mice to the effects of TNF in this experimental septic shock model. MAb 47 is a broad spectrum anti-TNF monoclonal antibody available from Peptide Technology Limited.
The protective effect of Peptide T was assessed after 12 hours. The results in the following Table XI were obtained.

TABLE XI

|   | A | B | C | D | E | F | G | H | Alive | Dose |
|---|---|---|---|---|---|---|---|---|-------|------|
| 1 | 0 | 0 | 0 | 0 | 0 | 0 | 0 | 0 | 0 | TNF |
| 2 | 0 | 4 | 10 | 4 | 2 | 3 | 6 | 10 | 14 | TNF + <An |
| 3 | 0 | 1 | 8 | 1 | 1 | 1 | 2 | 8 | 9 | TNF + 100µg PT |
| 4 | 1 | 1 | 5 | 3 | 1 | 1 | 0 | 5 | 7 | TNF + 10µg PT |
| 5 | 1 | 1 | 2 | 2 | 1 | 0 | 0 | 1 | 4 | TNF + 1µg PT |

Parameters: A Cold        E Diarrhoea
B Cool        F Discharge from Eyes
C Warm        G Touch Sensitive
D Ruffled        H Activity

EXAMPLE 14 - D-Thr-Thr-Tyr-D-Thr-NH$_2$ PROLONGS SURVIVAL OF SENSITISED MICE ADMINISTERED A LETHAL DOSE OF LPS

Meth A ascites tumour cells were injected (10$^6$ cells i.p.) into mice and allowed to grow. The Meth A tumour-bearing mice were thereby sensitised to the effects of TNF/LPS. The mice were administered with 50 µg LPS, formulated in phosphate-buffered saline (PBS), by injection i.p. and divided into three groups of ten. Treatment of each group was then as follows:

1. *Positive control.* 1 mg polymyxin B (PMB) in PBS injected i.p. (PMB is an inhibitor of LPS activity.)

2. *Negative control.* PBS injected i.p.

3. Compound under test. 1 mg Peptide T analogue 623 (D-Thr-Thr-Tyr-D-Thr-NH$_2$) in PBS injected i.p.

The results are shown in Figure 11. Survival of the mice treated with analogue 623 was at least as good as that of PMB-treated mice.

EXAMPLE 15 - PEPTIDE T INHIBITS ACTIVATION OF HUMAN NEUTROPHILS BY TNF AND LPS

*Neutrophils.* Neutrophils were isolated from whole blood from healthy blood donors. Separation was achieved by the rapid-single-step method of centrifugation of whole blood on FICOLL-HYPAQUE™. These were >99% viable and usually >98% pure.

*Peptides and TNF.* TNF was a recombinant product produced in *E. coli.* Peptide T and analogue 505 (D-Ser-Ser-D-Thr-Thr-D-Thr-Thr-Tyr-D-Thr) were resuspended in Hank's Balanced Salt Solution (HBSS).

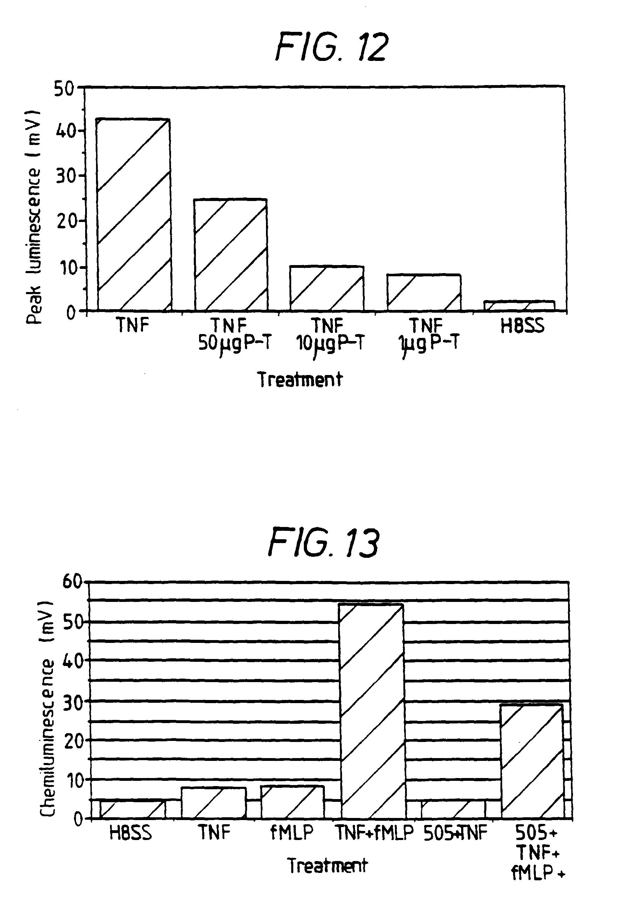

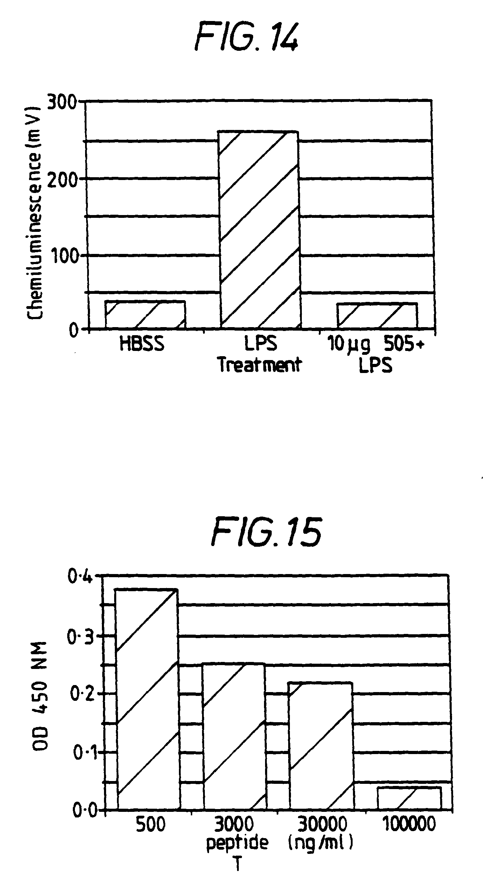

*Examination of Peptide T's and analogue 505's effect on stimuli-induced neutrophil (100μg) by chemiluminescence.* To 100μl of 500-1000U/ml of TNF was added 10μl of peptide and after 5 mm 100μl of $1x10^7$ neutrophils/ml was added. The cells further incubated at 37°C/20 min and then tested for chemiluminescence activity. In some cases an agonist such as fMLP ($10^{-7}$M) was added. Chemiluminescence was measured in a luminometer (LKB). The results for Peptide T are presented in Figure 12 as peak rate of CL release. Lucigenin-dependent chemiluminescence was measured: this represents superoxide measurement. In another set of experiments the effects of analogue 505 on the LPS-induced neutrophil chemiluminescence were examined. In this case the LPS was essentially substituted for the TNF. The concentration of LPS was 0.005μg/ml. The results are presented in Figures 13 and 14, which show respectively that analogue 505 (a) inhibits TNF priming and activation of human neutrophils and (b) inhibits LPS activation of human neutrophils.

EXAMPLE 16 - PEPTIDE T REDUCES TNF-MEDIATED PROTEOGLYCAN DEGRADATION AND REDUCES TNF'S ABILITY TO STIMULATE NEUTROPHIL-MEDIATED DEGRADATION OF CARTILAGE

(a) In one group of experiments various concentrations of peptide T were mixed with TNF and then added to $^{35}$S-labelled cartilage. The cartilage was cultured for 3 days at 37°C and on each day the supernatant was removed to determine the amount of degradation. The cartilage culture was replenished daily with TNF or peptide T-TNF.
(b) In another group of experiments the human neutrophils were treated with TNF or peptide T-TNF and then added to cartilage. The neutrophil mediated cartilage degradation determined.

RESULTS

1. Effects of peptide T on TNF-induced cartilage degradation

TABLE XII

| Effect of peptide T on TNF-induced cartilage proteoglycan degradation | | |
|---|---|---|
| Cartilage treatment | % Cumulative release (day 1,2,3) | |
| | Exp 1 | Exp 2 |
| TNF* | 45.5 | 23.1 |
| TNF + 1μg peptide T | 11.1 | -0.5 |
| TNF + 10μg peptide T | 3.2 | 1.7 |
| TNF + 100μg peptide T | 15.9 | 13.2 |

*100U of TNF

2. Effects of peptide T on TNF-primed neutrophil for proteoglycan degradation

Peptide T reduced the ability of TNF to stimulate neutrophil-mediated degradation of cartilage (Table XIII).

TABLE XIII

| The effect of peptide T on TNF priming of neutrophils for cartilage damage | | | |
|---|---|---|---|
| Treatment of neutrophils | % Cumulative release | | |
| | Exp 1 | Exp 2 | Exp 3 |
| TNF* | 17.4 | 6.8 | 5.3 |
| TNF + 10μg peptide T | -5.2 | 9.9 | 1.9 |
| TNF + 100μg peptide T | -0.8 | 0.9 | 0.6 |

* 100U or TNF

This example effectively gives good *in vitro* support to the anecdotal clinical evidence presented in Examples 13 and 14.

EXAMPLE 17 - PEPTIDE T REDUCES TISSUE FACTOR EXPRESSION INDUCED BY TNF

In acute systemic inflammation, for example in a septic shock patient, disseminated intravascular coagulation can be observed. During the course of the coagulation reaction, endothelial cells are involved as mediators of cell adhesion. One factor which is produced and which is involved in the coagulation cascade is tissue factor, also known as endothelial cell procoagulant activity; it is induced by TNF. In this example, it is demonstrated that Peptide T inhibits tissue factor expression by endothelial cells, thereby showing that Peptide T inhibits some of the pathology associated with acute inflammatory reactions.

*Methodology* Human umbilical vein endothelial cells were cultured essentially by the method of Bevilacqua *et al. Proc. Natl. Acad. Sci. USA* **83** 4533-4537 (1986) . Cell cultures were treated with TNFα at 3 μg/ml for 4 hours at 37° in the presence or absence of Peptide T. Tissue factor expression was measured by an ELISA, following absorbence at 450nm. The results are shown in Figure 15, from which it can be seen that increasing concentrations of Peptide T progressively reduce tissue factor expression.

**Claims**

**Claims for the following Contracting states AT, BE, CH, DE, DK, FR, GB, GR, IE, IT, LU, MC, NL, PT, SE**

1. Use of a linear or cyclic peptide of General Formula 1:

    I-A-B-C-D-E-F-G-H-II        (General Formula 1, SEQ ID NO 1)

    wherein

    A is Ala, Gly, Val, Ser, Thr or absent,
    B is Ala, Gly, Val, Ser, Thr or absent,
    C is Ser, Thr or absent,
    D is Ser, Thr, Asn, Glu, Arg, Ile, Leu or absent,
    E is Ser, Thr, Asp or absent,
    F is Thr, Ser, Asn, Arg, Gln, Lys, Trp or absent,
    G is Tyr or absent,
    H is Thr, Arg, Gly, Met, Met(O), Cys, Thr, Gly or absent,
    and
    I is Cys or absent
    II is Cys, an amide group, substituted amide group, an ester group or absent,

    and wherein the peptide comprises the amino acid sequence

    -Thr-Thr-Asn-Tyr-Thr-        (SEQ ID NO 3).

one or more of the amino acids optionally being substituted by a monomeric or polymeric carbohydrate or derivative thereof, such substitution being accomplished through hydroxyl and/or amino and/or amido groups of the amino acids,

or a pharmaceutically acceptable salt thereof, in the manufacture of a medicament for treating or preventing inflammatory bowel disease.

2. Use of a linear or cyclic peptide which is

1. D-Ala-Ser-Thr-Thr-Thr-Asn-Tyr-Thr-NH$_2$;
2. Ala-Ser-Thr-Thr-Thr-Asn-Tyr-Thr        (SEQ ID NO 5);
3. D-Ala-Ser-Thr-Thr-Thr-Asn-Tyr-Thr;
4. D-Ala-Ala-Ser-Ser-Ser-Asn-Tyr-Met;
5. Thr-Asp-Asn-Tyr-Thr        (SEQ ID NO 6);
6. Thr-Thr-Ser-Tyr-Thr        (SEQ ID NO 7);
7. Thr-Thr-Asn-Tyr-Thr        (SEQ ID NO 8);
8. D-Thr-Thr-Tyr-D-Thr;
9. D-Ala-Ser-D-Thr-Thr-D-Thr-Asn-Tyr-D-Thr-NH$_2$;
or
10. D-Ser-Ser-D-Thr-Thr-D-Thr-Thr-Tyr-D-Thr-NH$_2$;

or a derivative or salt thereof in the manufacture of a medicament for treating or preventing inflammatory bowel disease.

3. The use as claimed in claim 1 or claim 2, wherein at least one of the hydroxyl groups on a Ser, Thr or Tyr residue is derivatised into an ester or ether compound.

4. The use as claimed in any one of claims 1 to 3, wherein at least one of the amino acids is a substituted N-alkyl amino acid.

5. The use as claimed in any one of claims 1 to 4, wherein at least one of the amino acids is substituted with a monomeric or polymeric carbohydrate, or a derivative thereof, the substitution(s) being accomplished through hydroxyl- and/or amino- and/or amido-groups of the amino acids.

6. The use as claimed in any one of claims 1 to 5, wherein the peptide is D-Ala-Ser-Thr-Thr-Thr-Asn-Tyr-Thr-NH$_2$, or a derivative or salt thereof.

7. A peptide conjugate comprising a peptide as defined in any one of claims 1 to 6 conjugated to a peptide, protein or other appropriate carrier and/or an excipient.

8. A linear or cyclic peptide of General Formula 1:

I-A-B-C-D-E-F-G-H-II        (General Formula 1, SEQ ID NO 1)

wherein

A is Ala, Gly, Val, Ser, Thr or absent,
B is Ala, Gly, Val, Ser, Thr or absent,
C is Ser, Thr or absent,
D is Ser, Thr, Asn, Glu, Arg, Ile, Leu or absent,
E is Ser, Thr, Asp or absent,
F is Thr, Ser, Asn, Arg, Gln, Lys, Trp or absent,
G is Tyr or absent,
H is Thr, Arg, Gly, Met, Met(O), Cys, Thr, Gly or absent,
I is Cys or absent,
II is Cys or absent,

and wherein the peptide comprises the amino acid sequence

-Thr-Thr-Asn-Tyr-Thr-        (SEQ ID NO 3).

one or more of the amino acids being substituted by a monomeric or polymeric carbohydrate or derivative thereof, such substitution being accomplished through hydroxyl and/or amino and/or amido groups of the amino acids, with the proviso that the peptide is not glycosylated prototype Peptide T, Thr-Thr-Asn(GlcNAc)-Tyr-Thr or Thr (Gal-NAc)-Thr-Thr-Asn-Tyr-Thr;
or a pharmaceutically acceptable salt thereof.

9. A pharmaceutical composition comprising a peptide as defined in claim 8 in conjunction with a pharmaceutically acceptable carrier.

**Claims for the following Contracting State : ES**

1. A process for the manufacture of a medicament for treating or preventing inflammatory bowel disease, which process comprises use of a linear or cyclic peptide of General Formula 1:

    I-A-B-C-D-E-F-G-H-II        (General Formula 1, SEQ ID NO 1)

    wherein

    A is Ala, Gly, Val, Ser, Thr or absent,
    B is Ala, Gly, Val, Ser, Thr or absent,
    C is Ser, Thr or absent,
    D is Ser, Thr, Asn, Glu, Arg, Ile, Leu or absent,
    E is Ser, Thr, Asp or absent,
    F is Thr, Ser, Asn, Arg, Gln, Lys, Trp or absent,
    G is Tyr or absent,
    H is Thr, Arg, Gly, Met, Met(O), Cys, Thr, Gly or absent,
    and
    I is Cys or absent
    II IS Cys, an amide group, substituted amide group, an ester group or absent,

    and wherein the peptide comprises the amino acid sequence

    -Thr-Thr-Asn-Tyr-Thr-        (SEQ ID NO 3).

    one or more of the amino acids optionally being substituted by a monomeric or polymeric carbohydrate or derivative thereof, such substitution being accomplished through hydroxyl and/or amino acid and/or amido groups of the amino acids,
    or a pharmaceutically acceptable salt thereof.

2. A process for the manufacture of a medicament for treating or preventing inflammatory bowel disease, which process comprises use of a linear or cyclic peptide which is

    1. D-Ala-Ser-Thr-Thr-Thr-Asn-Tyr-Thr-NH$_2$;
    2. Ala-Ser-Thr-Thr-Thr-Asn-Tyr-Thr        (SEQ ID NO 5);
    3. D-Ala-Ser-Thr-Thr-Thr-Asn-Tyr-Thr;
    4. D-Ala-Ala-Ser-Ser-Ser-Asn-Tyr-Met;
    5. Thr-Asp-Asn-Tyr-Thr        (SEQ ID NO 6);
    6. Thr-Thr-Ser-Tyr-Thr        (SEQ ID NO 7);
    7. Thr-Thr-Asn-Tyr-Thr        (SEQ ID NO 8);
    8. D-Thr-Thr-Tyr-D-Thr;
    9. D-Ala-Ser-D-Thr-Thr-D-Thr-Asn-Tyr-D-Thr-NH$_2$;
    or
    10. D-Ser-Ser-D-Thr-Thr-D-Thr-Thr-Tyr-D-Thr-NH$_2$;

    or a derivative or salt thereof.

**3.** A process as claimed in claim 1 or claim 2, wherein at least one or the hydroxyl groups on a Ser, Thr or Tyr residue is derivatised into an ester or ether compound.

**4.** A process as claimed in any one of claims 1 to 3, wherein at least one of the amino acids is a substituted N-alkyl amino acid.

**5.** A process as claimed in any one of claims 1 to 4, wherein at least one of the amino acids is substituted with a monomeric or polymeric carbohydrate, or a derivative thereof, the substitution(s) being accomplished through hydroxyl- and/or amino- and/or amido-groups of the amino acids.

**6.** A process as claimed in any one of claims 1 to 5, wherein the peptide is D-Ala-Ser-Thr-Thr-Thr-Asn-Tyr-Thr-NH$_2$, or a derivative or salt thereof.

**7.** A peptide conjugate comprising a peptide as defined in any one of claims 1 to 6 conjugated to a peptide, protein or other appropriate carrier and/or an excipient.

**8.** A linear or cyclic peptide of General Formula 1:

I-A-B-C-D-E-F-G-H-II            (General Formula 1, SEQ ID NO 1)

wherein

A is Ala, Gly, Val, Ser, Thr or absent,
B is Ala, Gly, Val, Ser, Thr or absent,
C is Ser, Thr or absent,
D is Ser, Thr, Asn, Glu, Arg, Ile, Leu or absent,
E is Ser, Thr, Asp or absent,
F is Thr, Ser, Asn, Arg, Gln, Lys, Trp or absent,
G is Tyr or absent,
H is Thr, Arg, Gly, Met, Met(O), Cys, Thr, Gly or absent,
I is Cys or absent,
II is Cys or absent,

and wherein the peptide comprises the amino acid sequence

-Thr-Thr-Asn-Tyr-Thr-                (SEQ ID NO 3).

one or more of the amino acids being substituted by a monomeric or polymeric carbohydrate or derivative thereof, such substitution being accomplished through hydroxyl and/or amino and/or amido groups of the amino acids, with the proviso that the peptide is not glycosylated prototype Peptide T, Thr-Thr-Asn(GlcNAc)-Tyr-Thr or Thr (Gal-NAc)-Thr-Thr-Asn-Tyr-Thr;
or a pharmaceutically acceptable salt thereof.

**9.** A pharmaceutical composition comprising a peptide as defined in claim 8 in conjunction with a pharmaceutically acceptable carrier.

**10.** Use of a linear or cyclic peptide of General Formula 1:

I-A-B-C-D-E-F-G-H-II            (General Formula 1, SEQ ID NO 1)

wherein

A is Ala, Gly, Val, Ser, Thr or absent,
B is Ala, Gly, Val, Ser, Thr or absent,
C is Ser, Thr or absent,
D is Ser, Thr, Asn, Glu, Arg, Ile, Leu or absent,
E is Ser, Thr, Asp or absent,
F is Thr, Ser, Asn, Arg, Gln, Lys, Trp or absent,

G is Tyr or absent,

H is Thr, Arg, Gly, Met, Met(O), Cys, Thr, Gly or absent,
and
I is Cys or absent

II is Cys, an amide group, substituted amide group, an ester group or absent,

and wherein the peptide comprises the amino acid sequence

-Thr-Thr-Asn-Tyr-Thr-        (SEQ ID NO 3).

one or more of the amino acids optionally being substituted by a monomeric or polymeric carbohydrate or derivative thereof, such substitution being accomplished through hydroxyl and/or amino and/or amido groups of the amino acids,
or a pharmaceutically acceptable salt thereof, in the manufacture of a medicament for treating or preventing inflammatory bowel disease.

**11.** Use of a linear or cyclic peptide which is

1. D-Ala-Ser-Thr-Thr-Thr-Asn-Tyr-Thr-NH$_2$;
2. Ala-Ser-Thr-Thr-Thr-Asn-Tyr-Thr        (SEQ ID NO 5);
3. D-Ala-Ser-Thr-Thr-Thr-Asn-Tyr-Thr;
4. D-Ala-Ala-Ser-Ser-Ser-Asn-Tyr-Met;
5. Thr-Asp-Asn-Tyr-Thr        (SEQ ID NO 6);
6. Thr-Thr-Ser-Tyr-Thr        (SEQ ID NO 7);
7. Thr-Thr-Asn-Tyr-Thr        (SEQ ID NO 8);
8. D-Thr-Thr-Tyr-D-Thr;
9. D-Ala-Ser-D-Thr-Thr-D-Thr-Asn-Tyr-D-Thr-NH$_2$;
or
10. D-Ser-Ser-D-Thr-Thr-D-Thr-Thr-Tyr-D-Thr-NH$_2$;

or a derivative or salt thereof in the manufacture of a medicament for treating or preventing inflammatory bowel disease.

**12.** The use as claimed in claim 10 or claim 11, wherein at least one of the hydroxyl groups on a Ser, Thr or Tyr residue is derivatised into an ester or ether compound.

**13.** The use as claimed in any one of claims 10 to 12, wherein at least one of the amino acids is a substituted N-alkyl amino acid.

**14.** The use as claimed in any one of claims 10 to 13, wherein at least one of the amino acids is substituted with a monomeric or polymeric carbohydrate, or a derivative thereof, the substitution(s) being accomplished through hydroxyl- and/or amino- and/or amido-groups of the amino acids.

**15.** The use as claimed in any one of claims 10 to 14, wherein the peptide is D-Ala-Ser-Thr-Thr-Thr-Asn-Tyr-Thr-NH$_2$, or a derivative or salt thereof.

**Patentansprüche**

**Patentansprüche für folgende Vertragsstaaten : AT, BE, CH, DE, DK, FR, GB, GR, IE, IT, LU, MC, NL, PR, SE**

**1.** Verwendung eines linearen oder cyclischen Peptids der allgemeinen Formel (1):

I-A-B-C-D-E-F-G-H-II        (allgemeine Formel 1, SEQ ID NO 1)

worin

A Ala, Gly, Val, Ser, Thr ist oder fehlt,
B Ala, Gly, Val, Ser, Thr ist oder fehlt,

C Ser, Thr ist oder fehlt,

D Ser, Thr, Asn, Glu, Arg, Ile, Leu ist oder fehlt,

E Ser, Thr, Asp ist oder fehlt,

F Thr, Ser, Asn, Arg, Gln, Lys, Trp ist oder fehlt,

G Tyr ist oder fehlt,

H Thr, Arg, Gly, Met, Met(O), Cys, Thr, Gly ist oder fehlt,

und

I Cys ist oder fehlt

II Cys, eine Amidogruppe, eine substituierte Amidogruppe oder eine Estergruppe ist oder fehlt,

und worin das Peptid die Aminosäuresequenz

-Thr-Thr-Asn-Tyr-Thr- umfaßt        (SEQ ID NO 3),

und eine oder mehrere der Aminosäuren gegebenenfalls durch ein monomeres oder polymeres Kohlenhydrat oder ein Derivat davon substituiert sind, wobei diese Substitution über Hydroxyl- und/oder Amino- und/oder Amidogruppen der Aminosäuren erreicht wird,
oder eines pharmazeutisch annehmbaren Salzes davon zur Herstellung eines Arzneimittels zur Behandlung oder zur Verhinderung von entzündlichen Darmkrankheiten.

2. Verwendung eines linearen oder cyclischen Peptids, das ist:

1. D-Ala-Ser-Thr-Thr-Thr-Asn-Tyr-Thr-NH$_2$;
2. Ala-Ser-Thr-Thr-Thr-Asn-Tyr-Thr        (SEQ ID NO 5) ;
3. D-Ala-Ser-Thr-Thr-Thr-Asn-Tyr-Thr;
4. D-Ala-Ala-Ser-Ser-Ser-Asn-Tyr-Met;
5. Thr-Asp-Asn-Tyr-Thr        (SEQ ID NO 6) ;
6. Thr-Thr-Ser-Tyr-Thr        (SEQ ID NO 7) ;
7. Thr-Thr-Asn-Tyr-Thr        (SEQ ID NO 8) ;
8. D-Thr-Thr-Tyr-D-Thr;
9. D-Ala-Ser-D-Thr-Thr-D-Thr-Asn-Tyr-D-Thr-NH$_2$; oder
10. D-Ser-Ser-D-Thr-Thr-D-Thr-Thr-Tyr-D-Thr-NH$_2$;

oder eines Derivates oder Salzes davon zur Herstellung eines Medikamentes zur Behandlung oder Verhinderung entzündlicher Darmerkrankungen.

3. Verwendung nach Anspruch 1 oder Anspruch 2, dadurch gekennzeichnet, daß mindestens eine der Hydroxylgruppen an Ser, Thr oder Tyr in ein Ester- oder Etherderivat überführt ist.

4. Verwendung nach einem der Ansprüche 1 bis 3, dadurch gekennzeichnet, daß mindestens eine der Aminosäuren eine substituierte N-Alkyl-aminosäure ist.

5. Verwendung nach einem der Ansprüche 1 bis 4, dadurch gekennzeichnet, daß mindestens eine der Aminosäuren durch ein monomeres oder polymeres Kohlenhydrat oder ein Derivat davon substituiert ist, wobei die Substitution(en) über Hydroxyl- und/oder Amino- und/oder Amidogruppen der Aminosäuren bewirkt werden.

6. Verwendung nach einem der Ansprüche 1 bis 5, dadurch gekennzeichnet, daß das Peptid D-Ala-Ser-Thr-Thr-Thr-Asn-Tyr-Thr-NH$_2$, oder ein Derivat oder Salz davon ist.

7. Peptidkonjugat, das ein Peptid nach einem der Ansprüche 1 bis 6 an ein Peptid, Protein oder eine andere geeignete Trägersubstanz und/oder einen Arzneimittelträger konjugiert umfaßt.

8. Lineares oder cyclisches Peptid der allgemeinen Formel 1:

I-A-B-C-D-E-F-G-H-II        (allgemeine Formel 1, SEQ ID NO 1)

worin

A Ala, Gly, Val, Ser, Thr ist oder fehlt,

B Ala, Gly, Val, Ser, Thr ist oder fehlt,

C Ser, Thr ist oder fehlt,

D Ser, Thr, Asn, Glu, Arg, Ile, Leu ist oder fehlt,

E Ser, Thr, Asp ist oder fehlt,

F Thr, Ser, Asn, Arg, Gln, Lys, Trp ist oder fehlt,

G Tyr ist oder fehlt,

H Thr, Arg, Gly, Met, Met(O), Cys, Thr, Gly ist oder fehlt,

I Cys ist oder fehlt,

II Cys ist oder fehlt,

und worin das Peptid die Aminosäuresequenz umfaßt -Thr-Thr-Asn-Tyr-Thr (SEQ ID NO 3),
eine oder mehrere der Aminosäuren durch ein monomeres oder polymeres Kohlenhydrat oder ein Derivat davon substituiert sind, wobei die Substitution über Hydroxyl- und/oder Amino- und/oder Amidogruppen der Aminosäuren bewirkt wird,
mit der Maßgabe, daß das Peptid kein glycosyliertes Protoyp Peptid T, Thr-Thr-Asn(GlcNAc)-Tyr-Thr oder Thr(Gal-NAc)-Thr-Thr-Asn-Tyr-Thr ist;
oder ein pharmazeutisch annehmbares Salz davon.

**9.** Pharmazeutische Zusammensetzung, die ein Peptid nach Anspruch 8 zusammen mit einem pharmazeutisch annehmbaren Träger umfaßt.

**Patentansprüche für folgende Vertragsstaaten : ES**

**1.** Verfahren zur Herstellung eines Arzneimittels zur Behandlung oder Verhinderung entzündlicher Darmkrankheiten, wobei das Verfahren umfaßt die Verwendung eines linearen oder cyclischen Peptids der allgemeinen Formel (1):

I-A-B-C-D-E-F-G-H-II (allgemeine Formel 1, SEQ ID NO 1)

worin

A Ala, Gly, Val, Ser, Thr ist oder fehlt,
B Ala, Gly, Val, Ser, Thr ist oder fehlt,
C Ser, Thr ist oder fehlt,
D Ser, Thr, Asn, Glu, Arg, Ile, Leu ist oder fehlt,
E Ser, Thr, Asp ist oder fehlt,
F Thr, Ser, Asn, Arg, Gln, Lys, Trp ist oder fehlt,
G Tyr ist oder fehlt,
H Thr, Arg, Gly, Met, Met(O), Cys, Thr, Gly ist oder fehlt,
und
I Cys ist oder fehlt
II Cys, eine Amidogruppe, eine substituierte Amidogruppe oder eine Estergruppe ist oder fehlt,

und worin das Peptid die Aminosäuresequenz

-Thr-Thr-Asn-Tyr-Thr- umfaßt (SEQ ID NO 3),

eine oder mehrere der Aminosäuren gegebenenfalls durch ein monomeres oder polymeres Kohlenhydrat oder ein Derivat davon substituiert ist (sind), wobei diese Substitution über Hydroxyl- und/oder Amino und/oder Amidogruppen der Aminosäuren bewirkt wird,
oder eines pharmazeutisch annehmbaren Salzes davon.

**2.** Verfahren zur Herstellung eines Arzneimittels zur Behandlung oder Verhinderung von Darmkrankheiten, wobei das Verfahren umfaßt die Verwendung eines linearen oder cyclischen Peptids, das ist

1. D-Ala-Ser-Thr-Thr-Thr-Asn-Tyr-Thr-NH$_2$;
2. Ala-Ser-Thr-Thr-Thr-Asn-Tyr-Thr (SEQ ID NO 5) ;
3. D-Ala-Ser-Thr-Thr-Thr-Asn-Tyr-Thr;

4. D-Ala-Ala-Ser-Ser-Ser-Asn-Tyr-Met;

5. Thr-Asp-Asn-Tyr-Thr    (SEQ ID NO 6) ;

6. Thr-Thr-Ser-Tyr-Thr    (SEQ ID NO 7) ;

7. Thr-Thr-Asn-Tyr-Thr    (SEQ ID NO 8) ;

8. D-Thr-Thr-Tyr-D-Thr;

9. D-Ala-Ser-D-Thr-Thr-D-Thr-Asn-Tyr-D-Thr-NH$_2$; oder

10. D-Ser-Ser-D-Thr-Thr-D-Thr-Thr-Tyr-D-Thr-NH$_2$;

oder eines Derivates oder Salzes davon.

3. Verfahren nach Anspruch 1 oder Anspruch 2, dadurch gekennzeichnet, daß mindestens eine der Hydroxylgruppen an einem Ser-, Thr- oder Tyr-Rest zu einem Ester oder einem Ether derivatisiert ist.

4. Verfahren nach einem der Ansprüche 1 bis 3, dadurch gekennzeichnet, daß mindestens eine der Aminosäuren eine substituierte N-Alkyl-aminosäure ist.

5. Verfahren nach einem der Ansprüche 1 bis 4, dadurch gekennzeichnet, daß mindestens eine der Aminosäuren durch ein monomeres oder polymeres Kohlenhydrat oder ein Derivat davon substituiert ist, wobei die Substitution(en) über Hydroxy- und/oder Amino- und/oder Amidogruppen der Aminosäuren bewirkt wird.

6. Verfahren nach einem der Ansprüche 1 bis 5, dadurch gekennzeichnet, daß das Peptid D-Ala-Ser-Thr-Thr-Thr-Asn-Tyr-Thr-NH$_2$ oder ein Derivat oder Salz davon ist.

7. Peptidkonjugat nach einem der Ansprüche 1 bis 6, das mit einem Peptid, Protein oder einer anderen geeigneten Trägersubstanz und/oder einem Arzneimittelträger konjugiert ist.

8. Lineares oder cyclisches Peptid der allgemeinen Formel 1:

I-A-B-C-D-E-F-G-H-II    (allgemeine Formel 1, SEQ ID NO 1)

worin

A Ala, Gly, Val, Ser, Thr ist oder fehlt,
B Ala, Gly, Val, Ser, Thr ist oder fehlt,
C Ser, Thr ist oder fehlt,
D Ser, Thr, Asn, Glu, Arg, Ile, Leu ist oder fehlt,
E Ser, Thr, Asp ist oder fehlt,
F Thr, Ser, Asn, Arg, Gln, Lys, Trp ist oder fehlt,
G Tyr ist oder fehlt,
H Thr, Arg, Gly, Met, Met(O), Cys, Thr, Gly ist oder fehlt,
I Cys ist oder fehlt,
II Cys ist oder fehlt,

und worin das Peptid die Aminosäuresequenz umfaßt

-Thr-Thr-Asn-Tyr-Thr    (SEQ ID NO 3),

eine oder mehrere der Aminosäuren durch ein monomeres oder polymeres Kohlenhydrat oder ein Derivat davon substituiert sind, wobei die Substitution über Hydroxyl- und/oder Amino- und/oder Amidogruppen der Aminosäuren bewirkt wird,
mit der Maßgabe, daß das Peptid kein glycosyliertes Protoyp Peptid T, Thr-Thr-Asn(GlcNAc)-Tyr-Thr oder Thr(Gal-NAc)-Thr-Thr-Asn-Tyr-Thr ist;
oder ein pharmazeutisch annehmbares Salz davon.

9. Pharmazeutische Zusammensetzung, die ein Peptid nach Anspruch 1 zusammen mit einem pharmazeutisch annehmbaren Träger umfaßt.

10. Verwendung eines linearen oder cyclischen Peptids der allgemeinen Formel (1):

I-A-B-C-D-E-F-G-H-II          (allgemeine Formel 1, SEQ ID NO 1)

worin

A Ala, Gly, Val, Ser, Thr ist oder fehlt,
B Ala, Gly, Val, Ser, Thr ist oder fehlt,
C Ser, Thr ist oder fehlt,
D Ser, Thr, Asn, Glu, Arg, Ile, Leu ist oder fehlt,
E Ser, Thr, Asp ist oder fehlt,
F Thr, Ser, Asn, Arg, Gln, Lys, Trp ist oder fehlt,
G Tyr ist oder fehlt,
H Thr, Arg, Gly, Met, Met(O), Cys, Thr, Gly ist oder fehlt,
und
I Cys ist oder fehlt
II Cys, eine Amidogruppe, eine substituierte Amidogruppe oder eine Estergruppe ist oder fehlt,

und worin das Peptid die Aminosäuresequenz

-Thr-Thr-Asn-Tyr-Thr- umfaßt          (SEQ ID NO 3),

und eine oder mehrere der Aminosäuren gegebenenfalls durch ein monomeres oder polymeres Kohlenhydrat oder ein Derivat davon substituiert sind, wobei diese Substitution über Hydroxyl- und/oder Amino- und/oder Amidogruppen der Aminosäuren bewirkt wird,
oder eines pharmazeutisch annehmbaren Salzes davon zur Herstellung eines Arzneimittels zur Behandlung oder zur Verhniderung von entzündlichen Darmkrankheiten.

**11.** Verwendung eines linearen oder cyclischen Peptids, das ist:

1. D-Ala-Ser-Thr-Thr-Thr-Asn-Tyr-Thr-NH$_2$;
2. Ala-Ser-Thr-Thr-Thr-Asn-Tyr-Thr          (SEQ ID NO 5) ;
3. D-Ala-Ser-Thr-Thr-Thr-Asn-Tyr-Thr;
4. D-Ala-Ala-Ser-Ser-Ser-Asn-Tyr-Met;
5. Thr-Asp-Asn-Tyr-Thr          (SEQ ID NO 6) ;
6. Thr-Thr-Ser-Tyr-Thr          (SEQ ID NO 7) ;
7. Thr-Thr-Asn-Tyr-Thr          (SEQ ID NO 8) ;
8. D-Thr-Thr-Tyr-D-Thr;
9. D-Ala-Ser-D-Thr-Thr-D-Thr-Asn-Tyr-D-Thr-NH$_2$; oder
10. D-Ser-Ser-D-Thr-Thr-D-Thr-Thr-Tyr-D-Thr-NH$_2$;

oder eines Derivates oder Salzes davon zur Herstellung eines Medikamentes zur Behandlung oder Verhinderung entzündlicher Darmkrankheiten.

**12.** Verwendung nach Anspruch 10 oder Anspruch 11, dadurch gekennzeichnet, daß mindestens eine der Hydroxylgruppen an Ser, Thr oder Tyr in ein Ester- oder Etherderivat überführt ist.

**13.** Verwendung nach einem der Ansprüche 10 bis 12, dadurch gekennzeichnet, daß mindestens eine der Aminosäuren eine substituierte N-Alkyl-aminosäure ist.

**14.** Verwendung nach einem der Ansprüche 10 bis 13, dadurch gekennzeichnet, daß mindestens eine der Aminosäuren durch ein monomeres oder polymeres Kohlenhydrat oder ein Derivat davon substituiert ist, wobei die Substitution(en) über Hydroxyl- und/oder Amino- und/oder Amidogruppen der Aminosäuren bewirkt werden.

**15.** Verwendung nach einem der Ansprüche 10 bis 14, dadurch gekennzeichnet, daß das Peptid D-Ala-Ser-Thr-Thr-Thr-Asn-Tyr-Thr-NH$_2$, oder ein Derivat oder Salz davon ist.

**Revendications**

**Revendications pour les États contractants suivants : AT, BE, CH, DE, DK, FR, GB, GR, IE, IT, LU, MC, NL, PT, SE**

1.  Utilisation d'un peptide linéaire ou cyclique de formule générale 1 :

    I-A-B-C-D-E-F-G-H-II        (Formule générale 1, SEQ ID n° 1)

    dans laquelle

      A est Ala, Gly, Val, Ser, Thr ou absent,
      B est Ala, Gly, Val, Ser, Thr ou absent,
      C est Ser, Thr ou absent,
      D est Ser, Thr, Asn, Glu, Arg, Ile, Leu ou absent,
      E est Ser, Thr, Asp ou absent,
      F est Thr, Ser, Asn, Arg, Gln, Lys, Trp ou absent,
      G est Tyr ou absent,
      H est Thr, Arg, Gly, Met, Met(O), Cys, Thr, Gly ou absent,
      et
      I est Cys ou absent
      II est Cys, un groupe amide, un groupe amide substitué, un groupe ester ou absent,

    et dans laquelle le peptide comprend la séquence d'acides aminés

      -Thr-Thr-Asn-Tyr-Thr-            (SEQ ID n° 3)

    un ou plusieurs des acides aminés étant éventuellement substitués par un glucide monomère ou polymère ou un dérivé de celui-ci, une telle substitution étant réalisée via les groupes hydroxyle et/ou amino et/ou amido des acides aminés,
    ou un sel pharmaceutiquement acceptable de celui-ci, dans la fabrication d'un médicament destiné au traitement ou à la prévention d'une maladie intestinale inflammatoire.

2.  Utilisation d'un peptide linéaire ou cyclique qui est

      1. D-Ala-Ser-Thr-Thr-Thr-Asn-Tyr-Thr-$NH_2$ ;
      2. Ala-Ser-Thr-Thr-Thr-Asn-Tyr-Thr        (SEQ ID n° 5) ;
      3. D-Ala-Ser-Thr-Thr-Thr-Asn-Tyr-Thr ;
      4. D-Ala-Ala-Ser-Ser-Ser-Asn-Tyr-Met ;
      5. Thr-Asp-Asn-Tyr-Thr        (SEQ ID n° 6) ;
      6. Thr-Thr-Ser-Tyr-Thr        (SEQ ID n° 7) ;
      7. Thr-Thr-Asn-Tyr-Thr        (SEQ ID n° 8) ;
      8. D-Thr-Thr-Tyr-D-Thr ;
      9. D-Ala-Ser-D-Thr-Thr-D-Thr-Asn-Tyr-D-Thr-$NH_2$ ;
      ou
      10. D-Ser-Ser-D-Thr-Thr-D-Thr-Thr-Tyr-D-Thr-$NH_2$ ;

    ou un dérivé ou un sel de celui-ci dans la fabrication d'un médicament destiné au traitement ou à la prévention d'une maladie intestinale inflammatoire.

3.  Utilisation telle que revendiquée dans la revendication 1 ou la revendication 2, dans laquelle au moins un des groupes hydroxyle sur un résidu Ser, Thr, ou Tyr est dérivé en un composé ester ou éther.

4.  Utilisation telle que revendiquée dans l'une quelconque des revendications 1 à 3, dans laquelle au moins un des acides aminés est un acide aminé N-alkyle substitué.

5.  Utilisation telle que revendiquée dans l'une quelconque des revendications 1 à 4, dans laquelle au moins un des acides aminés est substitué avec un glucide monomère ou polymère, ou un dérivé de celui-ci, la (les) substitution(s) étant réalisée(s) via les groupes hydroxyle et/ou amino et/ou amido des acides aminés.

6. Utilisation telle que revendiquée dans l'une quelconque des revendications 1 à 5, dans laquelle le peptide est D-Ala-Ser-Thr-Thr-Thr-Asn-Tyr-Thr-NH$_2$, ou un dérivé ou un sel de celui-ci.

7. Conjugué de peptide comprenant un peptide tel que défini dans l'une quelconque des revendications 1 à 6 conjugué à un peptide, protéine ou autre véhicule approprié et/ou un excipient.

8. Peptide linéaire ou cyclique de formule générale 1 :

   I-A-B-C-D-E-F-G-H-II          (Formule générale 1, SEQ ID n° 1)

   dans laquelle

   A est Ala, Gly, Val, Ser, Thr ou absent,
   B est Ala, Gly, Val, Ser, Thr ou absent,
   C est Ser, Thr ou absent,
   D est Ser, Thr, Asn, Glu, Arg, Ile, Leu ou absent,
   E est Ser, Thr, Asp ou absent,
   F est Thr, Ser, Asn, Arg, Gln, Lys, Trp ou absent,
   G est Tyr ou absent,
   H est Thr, Arg, Gly, Met, Met(O), Cys, Thr, Gly ou absent,
   I est Cys ou absent,
   II est Cys ou absent,

   et dans laquelle le peptide comprend la séquence d'acides aminés

   -Thr-Thr-Asn-Tyr-Thr-          (SEQ ID n° 3)

   un ou plusieurs des acides aminés étant substitués par un glucide monomère ou polymère ou un dérivé de celui-ci, une telle substitution étant réalisée via les groupes hydroxyle et/ou amino et/ou amido des acides aminés, à condition que le peptide ne soit pas le Peptide prototype glycosylé T, Thr-Thr-Asn(GlcNAc)-Tyr-Thr ou Thr-(GalNAc)-Thr-Thr-Asn-Tyr-Thr ;
   ou un sel pharmaceutiquement acceptable de celui-ci.

9. Composition pharmaceutique comprenant un peptide tel que défini à la revendication 8 en combinaison avec un véhicule pharmaceutiquement acceptable.

**Revendications pour les Etats contractants suivants : ES**

1. Procédé pour la fabrication d'un médicament destiné au traitement ou à la prévention d'une maladie intestinale inflammatoire, procédé qui comprend l'utilisation d'un peptide linéaire ou cyclique de formule générale 1 :

   I-A-B-C-D-E-F-G-H-II          (Formule générale 1, SEQ ID n° 1)

   dans laquelle

   A est Ala, Gly, Val, Ser, Thr ou absent,
   B est Ala, Gly, Val, Ser, Thr ou absent,
   C est Ser, Thr ou absent,
   D est Ser, Thr, Asn, Glu, Arg, Ile, Leu ou absent,
   E est Ser, Thr, Asp ou absent,
   F est Thr, Ser, Asn, Arg, Gln, Lys, Trp ou absent,
   G est Tyr ou absent,
   H est Thr, Arg, Gly, Met, Met(O), Cys, Thr, Gly ou absent,
   et
   I est Cys ou absent
   II est Cys, un groupe amide, un groupe amide substitué, un groupe ester ou absent,

et dans laquelle le peptide comprend la séquence d'acides aminés

-Thr-Thr-Asn-Tyr-Thr-        (SEQ ID n° 3)

un ou plusieurs des acides aminés étant éventuellement substitués par un glucide monomère ou polymère ou un dérivé de celui-ci, une telle substitution étant réalisée via les groupes hydroxyle et/ou amino et/ou amido des acides aminés,
ou un sel pharmaceutiquement acceptable de celui-ci.

2. Procédé pour la fabrication d'un médicament destiné au traitement ou à la prévention d'une maladie intestinale inflammatoire, procédé qui comprend l'utilisation d'un peptide linéaire ou cyclique qui est

1. D-Ala-Ser-Thr-Thr-Thr-Asn-Tyr-Thr-NH$_2$ ;
2. Ala-Ser-Thr-Thr-Thr-Asn-Tyr-Thr        (SEQ ID n° 5) ;
3. D-Ala-Ser-Thr-Thr-Thr-Asn-Tyr-Thr ;
4. D-Ala-Ala-Ser-Ser-Ser-Asn-Tyr-Met ;
5. Thr-Asp-Asn-Tyr-Thr        (SEQ ID n° 6) ;
6. Thr-Thr-Ser-Tyr-Thr        (SEQ ID n° 7) ;
7. Thr-Thr-Asn-Tyr-Thr        (SEQ ID n° 8) ;
8. D-Thr-Thr-Tyr-D-Thr ;
9. D-Ala-Ser-D-Thr-Thr-D-Thr-Asn-Tyr-D-Thr-NH$_2$ ;
ou
10. D-Ser-Ser-D-Thr-Thr-D-Thr-Thr-Tyr-D-Thr-NH$_2$ ;

ou un dérivé ou un sel de celui-ci.

3. Procédé tel que revendiqué dans la revendication 1 ou la revendication 2, dans lequel au moins un des groupes hydroxyle sur un résidu Ser, Thr, ou Tyr est dérivé en un composé ester ou éther.

4. Procédé tel que revendiqué dans l'une quelconque des revendications 1 à 3, dans lequel au moins un des acides aminés est un acide aminé N-alkyle substitué.

5. Procédé tel que revendiqué dans l'une quelconque des revendications 1 à 4, dans lequel au moins un des acides aminés est substitué avec un glucide monomère ou polymère, ou un dérivé de celui-ci, la (les) substitution(s) étant réalisée(s) via les groupes hydroxyle et/ou amino et/ou amido des acides aminés.

6. Procédé tel que revendiqué dans l'une quelconque des revendications 1 à 5, dans lequel le peptide est D-Ala-Ser-Thr-Thr-Thr-Asn-Tyr-Thr-NH$_2$, ou un dérivé ou un sel de celui-ci.

7. Conjugué de peptide comprenant un peptide tel que défini dans l'une quelconque des revendications 1 à 6 conjugué à un peptide, protéine ou autre véhicule approprié et/ou un excipient.

8. Peptide linéaire ou cyclique de formule générale 1 :

I-A-B-C-D-E-F-G-H-II        (Formule générale 1, SEQ ID n° 1)

dans laquelle

A est Ala, Gly, Val, Ser, Thr ou absent,
B est Ala, Gly, Val, Ser, Thr ou absent,
C est Ser, Thr ou absent,
D est Ser, Thr, Asn, Glu, Arg, Ile, Leu ou absent,
E est Ser, Thr, Asp ou absent,
F est Thr, Ser, Asn, Arg, Gln, Lys, Trp ou absent,
G est Tyr ou absent,
H est Thr, Arg, Gly, Met, Met(O), Cys, Thr, Gly ou absent,
I est Cys ou absent,
II est Cys ou absent,

et dans laquelle le peptide comprend la séquence d'acides aminés

-Thr-Thr-Asn-Tyr-Thr-       (SEQ ID n° 3)

un ou plusieurs des acides aminés étant substitués par un glucide monomère ou polymère ou un dérivé de celui-ci, une telle substitution étant réalisée via les groupes hydroxyle et/ou amino et/ou amido des acides aminés, à condition que le peptide ne soit pas le Peptide prototype glycosylé T, Thr-Thr-Asn(GlcNAc)-Tyr-Thr ou Thr-(Gal-NAc)-Thr-Thr-Asn-Tyr-Thr ; ou un sel pharmaceutiquement acceptable de celui-ci.

9. Composition pharmaceutique comprenant un peptide tel que défini à la revendication 8 en combinaison avec un véhicule pharmaceutiquement acceptable.

10. Utilisation d'un peptide linéaire ou cyclique de formule générale 1 :

I-A-B-C-D-E-F-G-H-II        (Formule générale 1, SEQ ID n° 1)

dans laquelle

A est Ala, Gly, Val, Ser, Thr ou absent,
B est Ala, Gly, Val, Ser, Thr ou absent,
C est Ser, Thr ou absent,
D est Ser, Thr, Asn, Glu, Arg, Ile, Leu ou absent,
E est Ser, Thr, Asp ou absent,
F est Thr, Ser, Asn, Arg, Gln, Lys, Trp ou absent,
G est Tyr ou absent,
H est Thr, Arg, Gly, Met, Met(O), Cys, Thr, Gly ou absent,
et
I est Cys ou absent
II est Cys, un groupe amide, un groupe amide substitué, un groupe ester ou absent,

et dans laquelle le peptide comprend la séquence d'acides aminés

-Thr-Thr-Asn-Tyr-Thr-       (SEQ ID n° 3)

un ou plusieurs des acides aminés étant éventuellement substitués par un glucide monomère ou polymère ou un dérivé de celui-ci, une telle substitution étant réalisée via les groupes hydroxyle et/ou amino et/ou amido des acides aminés, ou un sel pharmaceutiquement acceptable de celui-ci, dans la fabrication d'un médicament destiné au traitement ou à la prévention d'une maladie intestinale inflammatoire.

11. Utilisation d'un peptide linéaire ou cyclique qui est

1. D-Ala-Ser-Thr-Thr-Thr-Asn-Tyr-Thr-NH$_2$ ;
2. Ala-Ser-Thr-Thr-Thr-Asn-Tyr-Thr       (SEQ ID n° 5) ;
3. D-Ala-Ser-Thr-Thr-Thr-Asn-Tyr-Thr ;
4. D-Ala-Ala-Ser-Ser-Ser-Asn-Tyr-Met ;
5. Thr-Asp-Asn-Tyr-Thr       (SEQ ID n° 6) ;
6. Thr-Thr-Ser-Tyr-Thr       (SEQ ID n° 7) ;
7. Thr-Thr-Asn-Tyr-Thr       (SEQ ID n° 8) ;
8. D-Thr-Thr-Tyr-D-Thr ;
9. D-Ala-Ser-D-Thr-Thr-D-Thr-Asn-Tyr-D-Thr-NH$_2$ ;
ou
10. D-Ser-Ser-D-Thr-Thr-D-Thr-Thr-Tyr-D-Thr-NH$_2$ ;

ou un dérivé ou un sel de celui-ci dans la fabrication d'un médicament destiné au traitement ou à la prévention d'une maladie intestinale inflammatoire.

**12.** Utilisation telle que revendiquée dans la revendication 10 ou la revendication 11, dans laquelle au moins un des groupes hydroxyle sur un résidu Ser, Thr, ou Tyr est dérivé en un composé ester ou éther.

**13.** Utilisation telle que revendiquée dans l'une quelconque des revendications 10 à 12, dans laquelle au moins un des acides aminés est un acide aminé N-alkyle substitué.

**14.** Utilisation telle que revendiquée dans l'une quelconque des revendications 10 à 13, dans laquelle au moins un des acides aminés est substitué avec un glucide monomère ou polymère, ou un dérivé de celui-ci, la (les) substitution(s) étant réalisée(s) via les groupes hydroxyle et/ou amino et/ou amido des acides aminés.

**15.** Utilisation telle que revendiquée dans l'une quelconque des revendications 10 à 14, dans laquelle le peptide est D-Ala-Ser-Thr-Thr-Thr-Asn-Tyr-Thr-NH$_2$, ou un dérivé ou un sel de celui-ci.

## FIG. 1

## FIG. 2a

## FIG. 2b

## FIG. 2c

## FIG.3

## FIG. 4

_-Peptide T_     _+Peptide T (Day 1)_

## FIG. 5

## FIG. 6

Day 1 ⬚ Day 2 ⬚ Day 3 ⬚ Day 4

## FIG. 7

## FIG.8

## FIG.9

## FIG. 10

## FIG. 11

## FIG. 12

## FIG. 13

## FIG. 14

## FIG. 15